(19) Europäisches Patentamt
European Patent Office
Office européen des brevets

(11) EP 2 571 893 B1

(12) **FASCICULE DE BREVET EUROPEEN**

(45) Date de publication et mention de la délivrance du brevet:
**10.08.2016 Bulletin 2016/32**

(21) Numéro de dépôt: **11724798.1**

(22) Date de dépôt: **17.05.2011**

(51) Int Cl.:
***C07K 7/64*** *(2006.01)* ***C07K 9/00*** *(2006.01)*
***G01N 33/68*** *(2006.01)*

(86) Numéro de dépôt international:
**PCT/IB2011/052162**

(87) Numéro de publication internationale:
**WO 2011/145055 (24.11.2011 Gazette 2011/47)**

(54) **NOUVEAUX COMPOSÉS CYCLODÉCAPEPTIDES POUR LEUR UTILISATION EN TANT QUE MÉDICAMENTS**

NEUARTIGE CYCLODECAPEPTIDVERBINDUNGEN ZUR VERWENDUNG ALS ARZNEIMEITTEL

NOVEL CYCLODECAPEPTIDE COMPOUNDS FOR USE AS DRUGS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.05.2010 FR 1002062**

(43) Date de publication de la demande:
**27.03.2013 Bulletin 2013/13**

(73) Titulaires:
- **Commissariat à l'Énergie Atomique et aux Énergies Alternatives
75015 Paris (FR)**
- **Université Grenoble Alpes
38400 Saint-Martin-d'Hères (FR)**

(72) Inventeurs:
- **DELANGLE, Pascale
F-38500 Voiron (FR)**
- **PUJOL, Anaïs
F-33510 AndernosLes Bains (FR)**
- **DUMY, Pascal
F-38000 Grenoble (FR)**
- **RENAUDET, Olivier
F-38830 St Pierre D'Allevard (FR)**
- **FERRAND, Michel
F-38140 Reaumont (FR)**

(74) Mandataire: **Gevers & Orès
41 avenue de Friedland
75008 Paris (FR)**

(56) Documents cités:
**WO-A1-2007/096517**

- **XU QINGCHAI ET AL: "A useful bicyclic topological decapeptide template for solution-phase combinatorial synthesis of tetrapodal libraries", TETRAHEDRON LETTERS, vol. 42, no. 41, 8 octobre 2001 (2001-10-08), pages 7261-7263, XP4304981, ISSN: 0040-4039**
- **PENG Z: "NMR conformational analysis on cyclic decapeptide template molecule", CANADIAN JOURNAL OF CHEMISTRY 1999 CA, vol. 77, no. 8, 1999, pages 1394-1404, XP002614964, ISSN: 0008-4042**
- **SINGH YASHVEER ET AL: "Synthetic peptide templates for molecular recognition: Recent advances and applications", CHEMBIOCHEM, vol. 7, no. 9, septembre 2006 (2006-09), pages 1298-1314, XP002614965, ISSN: 1439-4227**
- **ROUSSELOT-PAILLEY PIERRE ET AL: "Model peptides based on the binding loop of the copper metallochaperone Atx1: selectivity of the consensus sequence MxCxxC for metal ions Hg(II), Cu(I), Cd(II), Pb(II), and Zn(II).", INORGANIC CHEMISTRY 10 JUL 2006 LNKD-PUBMED:16813414, vol. 45, no. 14, 10 juillet 2006 (2006-07-10), pages 5510-5520, XP002614966, ISSN: 0020-1669 cité dans la demande**
- **BOTURYN DIDIER ET AL: "RAFT nano-constructs: surfing to biological applications", JOURNAL OF PEPTIDE SCIENCE, vol. 14, no. 2, février 2008 (2008-02), pages 224-240, XP002614967, ISSN: 1075-2617**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

SEQUENCE LISTING

<110> Commissariat à l'énergie atomique et aux énergies alternatives

<120> Nouveaux composés cyclodécapeptides et leurs applications

<130> F263-391

<160> 4

<170> PatentIn version 3.5

<210> 1
<211> 10
<212> PRT
<213> artificial sequence

<220>
<223> composé cyclodécapeptide de formule (I)

<220>
<221> MISC_FEATURE
<222> (1)..(1)
<223> X est un acide aminé présent sous sa forme dextrogyre ou lévogyre, et peut être substitué par un groupement Y1 choisi parmi -C(O)CHNL, -C(O)EL ou -NHEL dans lesquels L est un ligand biologique et E est un bras espaceur

<220>
<221> MISC_FEATURE
<222> (2)..(2)
<223> cet acide aminé cystéine peut être lié ou non par une liaison covalente à l'acide aminé cystéine en position 7, via leurs atomes de soufre

<220>
<221> MISC_FEATURE
<222> (3)..(3)
<223> X est un acide aminé présent sous sa forme dextrogyre ou lévogyre, et peut être substitué par un groupement Y3 choisi parmi -C(O)CHNL, -C(O)EL ou -NHEL dans lesquels L est un ligand biologique et E est un bras espaceur

<220>
<221> MISC_FEATURE
<222> (4)..(5)
<223> X est un acide aminé présent sous sa forme dextrogyre ou lévogyre

<220>
<221> MISC_FEATURE
<222> (4)..(5)
<223> X peut être substitué par un groupement sélectionné parmi -CO-marqueur, -NH-marqueur, -C(S)NH-marqueur, -SO2-marqueur, =CH-marqueur, -E'-marqueur, où E' est un bras espaceur

<220>

<221> MISC_FEATURE
<222> (4)..(5)
<223> la liaison X4-X5 est choisie parmi les liaisons (D)Pro-(L)X ou (L)Pro-(D)X', dans lesquelles X et X' sont des acides aminés

<220>
<221> MISC_FEATURE
<222> (6) .. (6)
<223> X est un acide aminé présent sous sa forme dextrogyre ou lévogyre, et peut être substitué par un groupement Y6 choisi parmi -C(O)CHNL, -C(O)EL ou -NHEL dans lesquels L est un ligand biologique et E est un bras espaceur

<220>
<221> MISC_FEATURE
<222> (7)..(7)
<223> cet acide aminé cystéine peut être lié ou non par une liaison covalente à l'acide aminé cystéine en position 2, via leurs atomes de soufre

<220>
<221> MISC_FEATURE
<222> (8)..(8)
<223> X est un acide aminé présent sous sa forme dextrogyre ou lévogyre, et peut être substitué par un groupement Y8 choisi parmi -C(O)CHNL, -C(O)EL ou -NHEL dans lesquels L est un ligand biologique et E est un bras espaceur

<220>
<221> MISC_FEATURE
<222> (9)..(10)
<223> X est un acide aminé présent sous sa forme dextrogyre ou lévogyre

<220>
<221> MISC_FEATURE
<222> (9)..(10)
<223> X peut être substitué par un groupement sélectionné parmi -CO-marqueur, -NH-marqueur, -C(S)NH-marqueur, -SO2-marqueur, =CH-marqueur, -E'-marqueur, où E' est un bras espaceur

<220>
<221> MISC_FEATURE
<222> (9)..(10)
<223> la liaison X9-X10 est choisie parmi les liaisons (D)Pro-(L)X ou (L)Pro-(D)X', dans lesquelles X et X' sont des acides aminés

<400> 1

```
Xaa Cys Xaa Xaa Xaa Xaa Cys Xaa Xaa Xaa
1               5                   10
```

<210> 2
<211> 10
<212> PRT
<213> artificial sequence

<220>
<223> composé cyclodécapeptide P1

<400> 2

```
Arg Cys Ser Pro Gly Ser Cys Trp Pro Gly
1               5                   10
```

<210> 3
<211> 10
<212> PRT
<213> artificial sequence

<220>
<223> composé cyclodécapeptide P2

<400> 3

```
Trp Cys Glu Pro Gly Glu Cys Asp Pro Gly
1               5                   10
```

<210> 4
<211> 10
<212> PRT
<213> artificial sequence

<220>
<223> composé cyclodécapeptide P3

<220>
<221> MISC_FEATURE
<222> (1) .. (1)
<223> cet acide aminé lysine est substitué par un groupement -C(O)CHNL

<220>
<221> MISC_FEATURE
<222> (2)..(2)
<223> cet acide aminé cystéine est lié par une liaison covalente avec l'acide aminé cystéine en position 7, via leurs atomes de soufre

<220>
<221> MISC_FEATURE
<222> (3)..(3)
<223> cet acide aminé lysine est substitué par un groupement -C(O)CHNL

<220>
<221> MISC_FEATURE
<222> (5)..(5)
<223> acide aminé D-lysine, qui peut être substitué par un groupement TRITC

<220>
<221> MISC_FEATURE
<222> (6)..(6)
<223> cet acide aminé lysine est substitué par un groupement -C(O)CHNL

<220>
<221> MISC_FEATURE
<222> (7)..(7)
<223> cet acide aminé cystéine est lié par une liaison covalente avec l'acide aminé cystéine en position 2, via leurs atomes de soufre

<220>
<221> MISC_FEATURE
<222> (8)..(8)
<223> cet acide aminé lysine est substitué par un groupement -C(O)CHNL

<400> 4

```
Lys Cys Lys Pro Lys Lys Cys Lys Pro Gly
1               5                   10
```

## Description

La présente invention concerne de nouveaux composés cyclodécapeptides de formule (I) pour leur utilisation en tant que médicaments, et plus particulièrement leur utilisation pour le diagnostic, la prévention et/ou le traitement de maladies neurodégénératives, telles que les maladies de Wilson et d'Alzheimer, et leur utilisation pour le diagnostic, la prévention et/ou le traitement d'intoxications avec des ions métalliques tels que les ions cuivre et mercure. Font également partie de l'invention des compositions pharmaceutiques comprenant en tant que principe actif au moins un composé de formule (I).

Les maladies liées à des dérèglements du transport du cuivre, comme la maladie de Wilson, conduisent à une accumulation du cuivre dans le foie, qui est l'unique organe capable de l'excréter. Ainsi, bien que le cuivre soit un élément essentiel à la vie, il peut, à l'état libre, induire des réactions d'oxydation de type Fenton, et par conséquent se révéler extrêmement toxique. Plus particulièrement, la maladie de Wilson est une maladie génétique liée à une déficience d'un transporteur du cuivre conduisant à une accumulation du cuivre dans différentes zones de l'organisme (jusqu'à 20 fois les taux normaux), qui se manifeste par des atteintes du foie et du système nerveux. Elle conduit à un dérèglement des concentrations physiologiques en cuivre chez l'individu qui se révèle par des troubles neurologiques et hépatiques sévères. Des troubles psychiques peuvent apparaître avec des modifications du caractère, conduisant à une hyperémotivité avec une grande labilité de l'humeur, des syndromes dépressifs et des états de psychose. La maladie de Wilson est induite par la mutation du gène ATP7B, qui code pour une protéine transmembranaire de type ATPase, intervenant dans le transport du cuivre, permettant la régulation de la concentration en cuivre, et son excrétion dans la bile. Si la protéine est déficiente, le métal s'accumule alors à l'intérieur des cellules. L'atteinte du foie précède en règle générale l'atteinte neurologique de quelques années. Les signes neurologiques ou psychiatriques concernent près de 50% des patients atteint de la maladie de Wilson. L'imagerie par résonance magnétique (IRM) montre des lésions de plusieurs structures cérébrales, même en l'absence de tout signe clinique et l'importance de celles-ci semble corréler avec le degré d'avancement de la maladie. Dans les cas gravissimes d'hépatites fulminantes ou dans les atteintes graves essentiellement hépatiques, une transplantation du foie peut être envisagée.

A l'heure actuelle, il existe des traitements dont l'objectif consiste à éradiquer la toxicité du cuivre accumulé dans l'organisme. Ces traitements doivent être suivis à vie, et ne doivent jamais être interrompus. Ils sont à base de médicaments chélateurs diminuant l'absorption du cuivre dans l'organisme, ou augmentant l'excrétion de ce métal. Les traitements doivent être soumis à une surveillance périodique, de façon à repérer l'apparition d'effets secondaires indésirables. Les traitements existants emploient différents principes actifs, tels que :

- la D-pénicillamine (Pen), qui augmente l'excrétion urinaire du cuivre (G. J. Brewer, DDT, 2005, 10, pp. 1103-1109). La D-pénicillamine a une efficacité reconnue et forme avec le cuivre (I) un complexe Cu(I)-Pen ayant une constante de stabilité de $10^{12}$ (M. Hefter et al., J. C. S., Chem. Commun., 1993, 1704-1706), toutefois elle présente de nombreux effets secondaires qui tendent à la faire remplacer par d'autres molécules. D'autre part, un certain nombre d'articles récents font état d'une aggravation de la maladie de Wilson par la D-pénicillamine et suggèrent de restreindre sa prescription dans cette indication ;
- la triéthylènetétramine (Trien), qui est un chélateur du cuivre souvent mieux toléré que la D-pénicillamine, et qui forme un complexe Cu(II)-Trien avec le cuivre (II) ayant une constante de stabilité de $10^{20}$ (R. M. Smith et al., 2001, NIST Critically Selected Stability Constants of Metal Complexes Database, NIST Standard Reference Database 46) ;
- l'anion de l'ammonium tétrathiomolybdate (TTM), absorbé avec l'alimentation, qui se fixe avec les ions cuivre dans le tube digestif, empêchant ainsi leur absorption ;
- le zinc active la production de protéines, les métallothionéines, qui vont fixer le cuivre dans les cellules de la paroi de l'intestin (entérocytes) empêchant le passage de cet ion dans la circulation sanguine (B. Sarkar, Chem. Rev., 1999, 99, 2535-2544).

Actuellement, ce sont les médicaments à base de D-pénicillamine, dont le mécanisme d'action est encore mal connu, qui sont le plus utilisés. Par sa fonction SH, la D-pénicillamine peut :

- chélater le cuivre et le zinc, mais aussi le mercure et le plomb, et augmenter leur excrétion urinaire,
- réduire les ponts disulfures de certaines molécules : collagène, fibres élastiques, immunoglobulines, et ainsi modifier leur activité biologique,
- se combiner à d'autres molécules soufrées, en particulier la cystéine, en formant des ponts disulfures.

Il apparaît effectivement que la présence d'atomes mous, tel que le soufre, permet une chélation plus efficace des ions métalliques dits « ions mous », tels que le cuivre Cu(I) et le mercure Hg(II).

Il existe également d'autres médicaments, dont l'action est à rapprocher de la D-pénicillamine en raison de la ressemblance de leurs propriétés pharmacologiques :

- le pyritinol, qui est une molécule symétrique formée de deux parties liées par un pont disulfure. Dans l'organisme, le pyritinol est coupé en deux molécules comportant chacune un groupement -SH. Toutefois, le pyritinol a été utilisé dans le traitement de la polyarthrite rhumatoïde avec des indications et des effets indésirables du même type que ceux de la D-pénicillamine,
- la tiopronine, qui est utilisée dans le traitement de fond de la polyarthrite rhumatoïde et de la lithiase cystinique.

Toutefois, les effets indésirables de la D-pénicillamine et des médicaments ayant un mode d'action similaire sont assez nombreux :

- cutanéo-muqueux précoces et peu graves : érythème, stomatite,
- cutanéo-muqueux tardifs et graves : toxicodermie, pemphigus, dermatomyosite,
- hématologiques : thrombopénie, leucopénie, agranulocytose, anémie hémolytique, justifiant la surveillance hématologique des malades traités,
- digestifs : agueusie,
- rénaux : protéinurie.

Les métaux sont également considérés comme des cibles thérapeutiques d'intérêt pour le diagnostic, la prévention et/ou le traitement de maladies neurodégénératives telle que la maladie d'Alzheimer, pour laquelle la dérégulation de l'homéostasie du zinc et du cuivre joue un rôle critique. Le cuivre Cu(II) est complexé et réduit en cuivre Cu(I) par la protéine APP et le peptide Aβ, le cuivre Cu(I) s'accumulant alors dans les plaques amyloïdes avec le fer et le zinc (E. Gaggelli *et al.,* 2006, 106, 1995-2044).

Le cuivre possède deux degrés d'oxydation stable dans des conditions différentes : le cuivre Cu(I) ayant un degré d'oxydation +I, stable en milieu réducteur, et le cuivre Cu(II) ayant un degré d'oxydation +II, stable en milieu oxygéné. Le cuivre présent dans les cellules humaines est principalement le cuivre Cu(I).

Des molécules, autres que la D-pénicillamine (Pen), peuvent également être utilisées pour chélater le cuivre *in vivo.* Il s'agit, par exemple, de l'acide 2,3-dimercaptosuccinique (DMSA) et de l'acide 2,3-dimercapto-1-propanesulfonique (DMPS) (O. Andersen, Chem. Rev., 1999, 99, 2683-2710), du 2,3-dimercaptopropanol (BAL), de la triéthylènetétramine (Trien), de l'anion de l'ammonium de tétrathiomolybdate (TTM) (G. J. Brewer et al., J. Hepatol., 2005, 42, S13-S21) et de l'acide éthylène-diamine-tétraacétique (EDTA), répondant aux formules semi-développées suivantes :

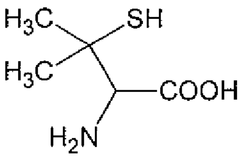

Pen

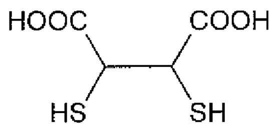

DMSA

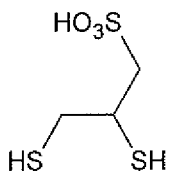

DMPS

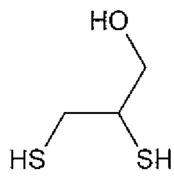

BAL

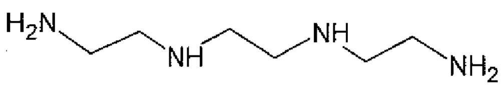

Trien

$MoS_4$ 2-

TTM

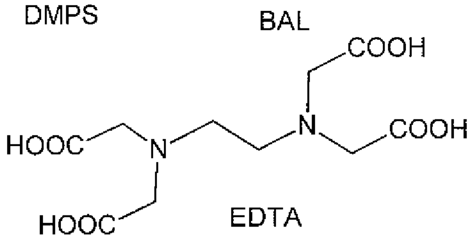

EDTA

Ces composés sont des agents chélatants connus du cuivre Cu(I) et/ou du cuivre Cu(II), bloquant l'absorption intestinale du cuivre. Toutefois, ces composés conduisent à des effets secondaires indésirables, et ne permettent pas le traitement de patients pour lesquels les maladies ont été détectées à un stade déjà avancé (détection non précoce), et pour lesquels il existe une accumulation intracellulaire importante du cuivre (B. Sarkar, Chem. Rev., 1999, 99, 2535-2534 ; G. J. Brewer et al., J. Hepatol., 2005, 42, S13-S21). De plus, certains agents chélatants, tel que l'EDTA, sont des agents chélatants très forts, chélateurs de nombreux ions métalliques, et dont l'un des principaux inconvénients est leur manque de sélectivité.

Les constantes de complexation apparentes de certains des agents chélatants connus (R. M. Smith et al., 2001, NIST Critically Selected Stability Constants of Metal Complexes Database, NIST Standard Reference Database 46) sont reportées dans le tableau I ci-après:

**Tableau I :**

| Log $K_{app}$ à T = 298 K (à pH = 7,4) | EDTA | Trien | Pen | BAL |
|---|---|---|---|---|
| Ca(II) | 7,8 | - | - | - |
| Cu(I) | - | - | 8,3 | - |
| Cu(II) | 16,0 | 16,0 | - | - |
| Zn(II) | 13,7 | 7,9 | 5,8 | 9,0 |
| Cd(II) | 13,7 | 6,6 | 7,6 | - |
| Hg(II) | 18,7 | 20,6 | 14,9 | 21,2 |
| Pb(II) | 15,2 | 6,3 | 9,2 | - |
| Sélectivité Cu/Zn | 2,3 | 8,1 | 2,5 | - |
| Sélectivité Hg/Zn | 5 | 12,7 | 9,1 | 12,2 |

La sélectivité entre deux métaux M/M' correspond à la sélectivité du ligand pour le métal M par rapport à celle du métal M', cette sélectivité étant égale à :

$$\log (K_{app}(M)/K_{app}(M')) = \log K_{app}(M) - \log K_{app}(M')$$

L'EDTA et la Trien sont des agents chélatants du cuivre Cu(II). L'EDTA est un agent chélatant hexadente très fort possédant des atomes donneurs azote et oxygène, dont l'un des principaux désavantages est le manque de sélectivité (il complexe très fortement tous les ions essentiels et toxiques cités dans le Tableau I). La Trien, quant à elle, est un agent chélatant polyamine très fort qui complexe fortement les ions métalliques mercure Hg(II) et cuivre Cu(II), et qui présente une relative sélectivité vis-à-vis des ions zinc Zn(II).

La Pen est un agent chélatant comportant une fonction thiol ainsi que des atomes donneurs azote et oxygène. La présence du groupement thiol permet d'atteindre une affinité relativement élevée avec l'ion toxique Hg(II), tout en étant sélectif vis-à-vis des ions zinc Zn(II). Toutefois, la sélectivité de la Pen pour les ions cuivre Cu(I) (par rapport aux ions zinc Zn(II)) reste faible.

Le BAL est un agent chélatant dithiol qui présente une affinité très élevée pour les ions mercure Hg(II), et probablement également pour les ions cuivre Cu(I).

Ainsi, il apparaît que l'introduction de fonctions thiols favorise la complexation des ions mous, tels que les ions mercure Hg(II) et cuivre Cu(I), par rapport aux autres ions.

Il subsiste néanmoins aujourd'hui un besoin en agents chélatants plus sélectifs, en particulier à l'égard du cuivre, et plus particulièrement du Cu(I) intracellulaire, et surtout moins toxiques, dont les effets secondaires seraient moins violents que ceux des molécules actuellement utilisées.

Les inventeurs ont trouvé de manière surprenante que les nouveaux composés de l'invention décrits ci-après apparaissent comme une meilleure alternative, en particulier en termes de sélectivité, par rapport aux molécules précédemment développées, pour le diagnostic, la prévention et/ou le traitement de maladies neurodégénératives et/ou d'intoxications avec des ions métalliques, et plus particulièrement avec des ions cuivre et mercure, ces composés étant notamment utiles pour le traitement de la maladie de Wilson (dont les patients présentent un excès de cuivre dans le foie) et pour le traitement de la maladie d'Alzheimer, ou pour détoxiquer le foie intoxiqué au mercure Hg(II).

Ainsi, la présente invention concerne de nouveaux composés cyclodécapeptides de formule (I) pour leur utilisation en tant que médicaments, notamment pour le diagnostic, la prévention et/ou le traitement de maladies neurodégénératives, telles que les maladies de Wilson et d'Alzheimer, et pour le diagnostic, la prévention et/ou le traitement d'intoxications avec des ions métalliques tels que les ions cuivre et mercure. Les composés de l'invention peuvent ainsi être utilisés pour le diagnostic et la prévention de maladies neurodégénératives chez des personnes présentant un risque plus important du fait de facteurs génétiques ou environnementaux.

Ainsi, le premier objet de la présente invention concerne les composés cyclodécapeptides répondant à la formule (I) suivante :

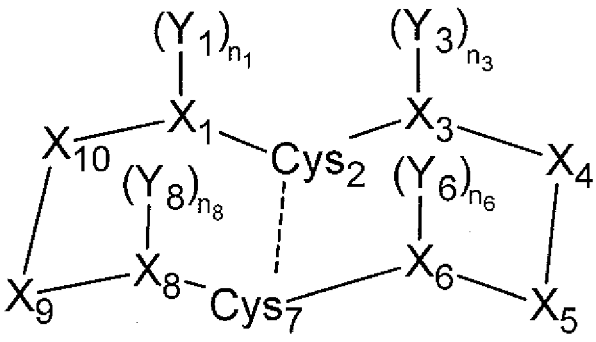

(I)

dans lesquels :

✔ les acides aminés cystéines $Cys_2$ et $Cys_7$ peuvent être liés ou non par une liaison covalente $Cys_2$-$Cys_7$ via leurs atomes de soufre,
✔ $X_1$, $X_3$, $X_4$, $X_5$, $X_6$, $X_8$, $X_9$, $X_{10}$, identiques ou différents, sont des acides aminés pouvant être présents sous la forme de deux énantiomères optiquement actif : l'énantiomère dextrogyre (D) ou l'énantiomère lévogyre (L),
✔ $n_1$, $n_3$, $n_6$, $n_8$, identiques ou différents, sont égaux à 0 ou 1,
✔ $Y_1$, $Y_3$, $Y_6$, $Y_8$, identiques ou différents, représentent des groupements-C(O)CHNL, -C(O)EL ou -NHEL, et de préférence un groupement-C(O)CHNL, dans lesquels L est un ligand biologique, et de préférence un ligand biologique de cellules hépatiques ou neuronales, sélectionné parmi les oses tels que le glucose, le galactose et le N-acétylgalactosamine, et E est un bras espaceur sélectionné parmi les polyols tels que le polyéthylène glycol ayant de préférence 1 à 8 motifs oxyéthylène OE, et les chaînes alkyles ayant 1 à 12 atomes de carbone, éventuellement substituées par un ou plusieurs substituants choisis parmi les chaînes alkyles ou alcoxy en $C_1$-$C_6$, les groupements -OH, -COOH, $-NO_2$, $-NH_2$, $-C(O)NH_2$, -SH ou les atomes d'halogène,
✔ en option, au moins un des acides aminés $X_4$, $X_5$, $X_9$, $X_{10}$, et/ou au moins un des groupements $Y_1$, $Y_3$, $Y_6$, $Y_8$, peut être substitué par un groupement sélectionné parmi : -CO-marqueur, -NH-marqueur, -C(S)NH-marqueur, $-SO_2$-marqueur, =CH-marqueur, -E'-marqueur, où E' est un bras espaceur sélectionné parmi le phényle, le triazole, l'oxadiazole, l'oxazole, l'imidazole, le thiadiazole, le pyrrole, le tétrazole, le furane, le thiophène, le pyrazole, la pyrazoline, la pyrazidine, le thiazole, l'isothiazole, la pyridine, la pyrimidine, la pipéridine, le pyranne, la pyrazine, la pyridazine et leurs dérivés, et

étant entendu que les liaisons $X_4$-$X_5$ et $X_9$-$X_{10}$, identiques ou différentes, sont choisies parmi les liaisons (D)Pro-(L)X ou (L)Pro-(D)X', dans lesquelles X et X' sont des acides aminés, de préférence choisis parmi la glycine, la lysine, le glutamate ou l'aspartate,
pour leur utilisation en tant que médicaments.

Selon un mode de réalisation avantageux, l'un au moins des acides aminés $X_1$, $X_3$, $X_6$, $X_8$ est une lysine. De manière alternative, l'un au moins des acides aminés $X_1$, $X_3$, $X_6$, $X_8$ est une lysine portant un groupement $Y_1$, $Y_3$, $Y_6$, $Y_8$.

Selon un autre mode de réalisation avantageux, le composé de formule (I) selon l'invention est un composé dans lequel au moins un des acides aminés $X_4$, $X_5$, $X_9$, $X_{10}$, et/ou au moins un des groupements $Y_1$, $Y_3$, $Y_6$, $Y_8$, est substitué par un groupement sélectionné parmi : -CO-marqueur, -NH-marqueur, -C(S)NH-marqueur, $-SO_2$-marqueur, =CH-marqueur, -E'-marqueur.

On entend par marqueur toute entité susceptible d'être détectée par des moyens appropriés, les marqueurs utilisés dans le cadre de l'invention correspondant typiquement aux marqueurs utilisés par l'homme de l'art dans le domaine de la biologie pour marquer des molécules d'intérêt biologiques, notamment dans le cadre de la réalisation de diagnostic, d'études galéniques, ou encore de suivi de la métabolisation de composés actifs. Le marquage peut être de nature directe, et dans ce cas le marqueur est qualifié de « marqueur direct » et présente au moins une propriété physique détectable, ou le marquage peut être de nature indirecte, et dans ce cas le marqueur est qualifié de « marqueur indirect » et est susceptible de réagir sélectivement avec une entité tierce, cette dernière pouvant soit présenter au moins une propriété physique détectable, comme par exemple un anticorps présentant une activité fluorescente, soit être investie dans un processus réactionnel à l'issu duquel une propriété physique pourra être détectée, comme par exemple lorsque le produit de dégradation de l'entité peut présenter au moins une propriété physique détectable telle que de la fluorescence. Le marquage indirect est souvent réalisé à l'aide d'anticorps ou de nanoparticules possédant une activité fluorescente. Dans ce cas, le marqueur indirect des composés de formule (I) possède une affinité pour l'entité tierce.

Ainsi, le marqueur de l'invention peut être soit une entité chimique de nature organique, soit une entité chimique

de nature inorganique, telle qu'un complexe ou un cristal, ce dernier pouvant éventuellement être enrobé d'une couche organique, cette entité chimique de nature inorganique étant généralement de taille suffisamment faible, typiquement à l'échelle nanométrique, pour ne pas perturber le système biologique dans lequel elle est introduite.

La propriété physique détectable, directement ou indirectement, peut être une réactivité spécifique vis-à-vis d'une source électromagnétique telle qu'un champ magnétique, comme par exemple par imagerie par résonance magnétique, ou vis-à-vis d'un rayonnement lumineux pouvant être focalisé, comme par exemple par imagerie par fluorescence avec les fluorophores, ou encore vis-à-vis d'un rayonnement nucléaire, comme par exemple à l'aide d'isotopes.

Les marqueurs les plus préférés sont les marqueurs directs, et plus particulièrement les fluorophores. Typiquement, il s'agit de fluorophores organiques ou de nanoparticules. Les fluorophores utilisés dans le cadre de l'invention peuvent être des composés fluorescents aromatiques dont les transitions $\pi$-$\pi$ sont caractérisées par des coefficients d'absorption molaires et des rendements quantiques de fluorescence élevées, lesdits fluorophores pouvant être choisis parmi la rhodamine, la fluorescéïne, la pyronine, la coumarine, la benzophénone, l'anthrone, la fluorénone, la pyridine, la quinoléine, l'acridine, le naphtalène, l'anthracène, la naphtacène, la pentacène, le xanthène et leurs dérivés.

Les différentes familles de marqueurs et les différentes techniques de détection associées sont connues de l'homme de l'art et décrites dans l'ouvrage Anti-Cancer Ag. in Med. Chem., 2008, 8, 497-522. Plus spécifiquement, il est possible de se référer aux fluorophores cités dans Cytometry Part A, 2006, 69A : 863-871, et aux nanoparticules mentionnées dans le document Anal. Bioanal. Chem., 2006, 384 : 620-630.

Ainsi, les composés marqués de formule (I) de l'invention peuvent être utilisés pour visualiser le cheminement desdits composés dans l'organisme, par luminescence.

Selon un autre mode de réalisation avantageux de l'invention, les composés de l'invention sont des composés dans lesquels $n_1$, $n_3$, $n_6$, $n_8$ = 0, lesdits composés répondant alors à la formule (Ia) suivante :

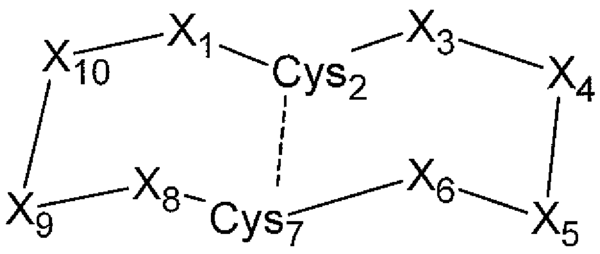

(Ia)

Un autre objet de l'invention concerne les composés de formule (I) selon l'invention, pour la préparation d'un médicament destiné au diagnostic, à la prévention et/ou au traitement de maladies neurodégénératives, et plus particulièrement pour le traitement des maladies de Wilson et d'Alzheimer.

Les composés de l'invention peuvent également être utilisés en tant qu'agents chélatants d'ions métalliques pour la préparation d'un médicament destiné au diagnostic, à la prévention et/ou au traitement d'intoxications avec des ions métalliques tels que les ions argent, cadmium, cobalt, cuivre, mercure, nickel, or, plomb et zinc, et plus particulièrement pour le traitement d'intoxications avec des ions mercure ou cuivre, tels que les ions cuivre Cu(I) intracellulaire, les intoxications par de tels ions conduisant généralement à des inflammations sévères, des déficiences rénales, des hémorragies, des troubles neurologiques sévères du système nerveux central ; on parle notamment d'hydrargie (ou d'hydrargyrisme) dans le cas d'intoxications par le mercure.

Il est à noter qu'une autre utilisation possible des composés de formule (I) selon l'invention, dans lesquels les acides aminés cystéines $Cys_2$ et $Cys_7$ ne sont pas liés par une liaison covalente, concerne leur utilisation *in vitro* en tant qu'agents dépolluants pour dépolluer les eaux contaminées, de préférence en milieu réducteur. La valeur du pH limite du milieu dépend de l'ion métallique à complexer. Ainsi, pour les ions Hg(II) et Cu(I) le pH du milieu réducteur est de préférence supérieur ou égal à 1, et pour les ions Zn(II), Pb(II) et Cd(II) le pH du milieu réducteur est de préférence supérieur ou égal à 4 ou 6.

Les composés de formule (I) de l'invention, dans lesquels les acides aminés cystéines $Cys_2$ et $Cys_7$ sont liés par une liaison covalente $Cys_2$-$Cys_7$ via leurs atomes de soufre, sont transformés en milieu réducteur en composés de formule (I), dans lesquels les acides aminés cystéines $Cys_2$ et $Cys_7$ sont libres (absence de liaison covalente entre les atomes de soufre des cystéines $Cys_2$ et $Cys_7$). Cette réaction de réduction permet la libération des fonctions thiols des cystéines $Cys_2$ et $Cys_7$ (qui étaient masquées par la liaison covalente $Cys_2$-$Cys_7$), une fois que les composés de formule (I) vectorisés dans l'organisme sont entrés dans les cellules ciblées. Les composés de formule (I), dans lesquels les acides aminés cystéines $Cys_2$ et $Cys_7$ sont liés par une liaison covalente $Cys_2$-$Cys_7$ via leurs atomes de soufre, peuvent donc être utilisés en tant que précurseurs pour la vectorisation dans l'organisme de composés de formule (I), dans lesquels les acides aminés cystéines $Cys_2$ et $Cys_7$ ne sont pas liés par une liaison covalente via leurs atomes de soufre.

L'agent réducteur permettant l'obtention des composés de formule (I), dans lesquels les acides aminés cystéines $Cys_2$ et $Cys_7$ sont libres, peut être une molécule porteuse d'une fonction thiol, telle que l'éthanedithiol (EDT), le glutathion (GSH), la cystéine et le dithiotréitol (DTT), ou une molécule porteuse d'une fonction phosphine telle que le tris(2-carboxyéthyl)phosphine (TCEP).

Ainsi, la libération des fonctions thiols des acides aminés cystéines $Cys_2$ et $Cys_7$ se fait par réduction *in vivo* dans l'organisme, par exemple dans les cellules hépatiques où le glutathion (GSH), qui est présent à environ 1 mM, peut jouer le rôle de réducteur.

Enfin, un dernier objet de l'invention concerne des compositions pharmaceutiques comprenant en tant que principe actif au moins un composé de formule (I) selon l'invention, et au moins un véhicule pharmaceutiquement acceptable. Lesdites compositions pharmaceutiques incluent aussi bien les compositions sous forme solide (comprimés, gélules, capsules, etc...), que les compositions sous forme liquide (solutions, suspensions ou émulsions), et incluent les excipients adaptés à une administration orale, topique ou parentérale. L'administration des composés ou des compositions selon l'invention est effectuée de préférence par voie orale ou par voie parentérale (intraveineuse en perfusion ou injection, notamment). Les doses de composés sont de préférence inférieures à 2 g de produit par jour, et varient selon la formulation sélectionnée, le mode d'administration et l'intoxication ou la maladie à traiter. D'autres facteurs tels que l'âge, le poids, la taille, le sexe, ainsi que certains paramètres biologiques (taux d'excrétion, association avec d'autres médicaments, allergies...) sont également à prendre en compte.

Outre les dispositions qui précèdent, l'invention comprend encore d'autres dispositions qui ressortiront du complément de description qui suit, qui se rapporte à des exemples mettant en évidence les propriétés de complexation, et plus particulièrement les propriétés de complexation du cuivre Cu(I), dans les cellules hépatiques, des composés de l'invention, ainsi qu'aux figures annexées dans lesquelles :

- la Figure 1 représente le dosage UV d'un composé $P^1$ selon l'invention par du $Cu(CH_3CN)PF_6$, dans une solution tampon phosphate à 20 mM, de pH = 7,4, à une température de 298 K,
- la Figure 2 représente le spectre de masse ES-MS d'un composé $P^1$ selon l'invention, en présence d'un équivalent de Cu(I),
- la Figure 3 montre l'évolution de la quantité de cuivre Cu(I) libre, détectée par du disulfonate bathocuproïne (BCS), et mesurée par absorption UV du complexe $Cu(BCS)_2$, dans une solution tampon phosphate à 20 mM, de pH = 7,4, et avec $[P^3] = [P^1] = [P^2] = 50\ \mu M$, $[Cu(I)] = 40\ \mu M$ et $[BCS] = 100\ \mu M$,
- la Figure 4 représente des images obtenues par microscopie de fluorescence (grossissement x 63) montrant l'évolution d'un composé **$P^3$-TRITC** selon l'invention (0,2 μm) dans des cellules HepG2, après 2 heures, 7 heures et 26 heures d'incubation,
- la Figure 5 représente des images obtenues par microscopie de fluorescence (grossissement x 63) montrant l'évolution d'un composé **$P^3$-TRITC** selon l'invention (0,2 μm) dans des cellules Can10, après 2 heures, 7 heures et 26 heures d'incubation,
- la Figure 6 représente des images obtenues par microscopie de fluorescence (grossissement x 63) montrant la localisation de la protéine ATP7B dans des hépatocytes WIF-B9, en conditions basales (image A) et en présence de 1 μM de cuivre Cu(I) (image B), et
- la Figure 7 représente des images obtenues par microscopie de fluorescence (grossissement x 63) réalisées en présence de 1 μM de cuivre Cu(I), en absence d'un composé **$P^3$** selon l'invention (images A) et en présence de 10 μM d'un composé **$P^3$** selon l'invention (images B).

## Exemples :

### A - Méthodes de caractérisation

#### 1/ Chromatographie en phase liquide à haute performance (HPLC)

La chromatographie HPLC est réalisée sur un système VWR muni de colonnes RP18 (L = 250 mm, Ø = 4,6 mm et p = 5 μm, pour la colonne analytique ; L = 250 mm, Ø = 50 mm et p = 10 μm, pour la colonne préparatoire).

Les débits utilisés sont de 1 mL/min pour la colonne analytique et de 75 mL/min pour la colonne préparatoire, avec une détection UV à 214 nm.

Les conditions d'élution sont les suivantes :

- solvant A : mélange eau/acide trifluoroacétique (TFA) (99,925/0,075), et
- solvant B : mélange acétonitrile ($CH_3CN$)/eau/acide trifluoroacétique (TFA) (90/10/0,1).

### 2/ Spectroscopie UV-visible

Les spectres UV-visible ont été enregistrés sur un spectrophotomètre Varian Cary 50.

### 3/ Spectrométrie de masse

Les spectres de masse ont été enregistrés sur un spectromètre de type LXQ THERMO SCIENTIFIC, équipé d'une source d'ionisation en mode électrospray (ESI).

### 4/ Microscopie de fluorescence

Les images de microscopie de fluorescence sont obtenues sur un microscope inversé AxioVert 200M (Carl Zeiss), équipé d'une lampe à vapeur de mercure NHBO 103 et d'une lampe halogène HAL 100W, ainsi que d'un dispositif de mesure de la fluorescence.

Les images sont réalisées avec un grossissement x 63.

## B - Synthèses

**Synthèses de composés cyclodécapeptides $P^1$ et $P^2$ répondant à la formule (Ia) de l'invention (les acides aminés $Cys_2$ et $Cys_7$ n'étant pas liés entre eux par une liaison covalente)** :

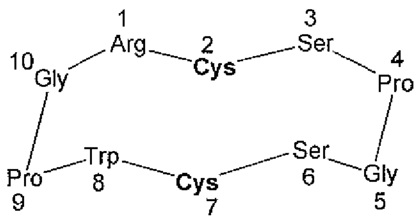

**$P^1$**

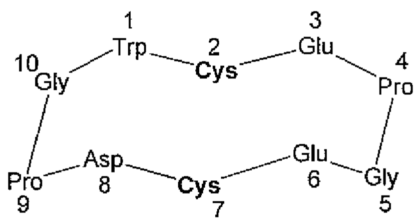

**$P^2$**

Les précurseurs linéaires protégés HArg(Pbf)-Cys(Trt)-Ser(tBu)-Pro-Gly-Ser(tBu)Cys(Trt)-Trp(Boc)Pro-Gly-OH et H-Trp(Boc)-Cys(Trt)-Glu(tBu)-Pro-Gly-Glu(tBu)-Cys(Trt)-Asp(tBu)-Pro-GlyOH ont été préparés par synthèse peptidique en phase solide sur une résine chlorure de chloro-2-trityle (substitution 0,5 mmol/g, 500 mg) par chimie Fmoc (synthèse peptidique sur support solide utilisant le 9-fluorénylméthoxycarbonyle comme groupement protecteur, R. Sheppard, J. Peptide Sci., 2003, 9 : 545-552). Les couplages sont réalisés par mélange des acides aminés N-$\alpha$-Fmoc-protégés (2 équivalents), avec du benzotriazol-1-yl-oxytripyrrolidinophosphonium (PyBOP) (2 équivalents) et du N,N-diisopropyléthylamine (DIEA) (6 équivalents), pendant 30 minutes. Après chaque couplage, la résine est traitée avec un mélange DMF/pyridine/$Ac_2O$ (v/v/v = 7/2/1) pour acétyler les groupements amino n'ayant pas réagi (2 x 5 minutes). La déprotection des groupements Fmoc est réalisée par traitement avec un mélange DMF/pipéridine (v/v = 4/1, 3 x 5 minutes). Le rendement obtenu pour chaque peptide est suivi par spectrométrie UV-visible ($\varepsilon^{300\ nm}$ = 7800 L.mol$^{-1}$.cm$^{-1}$ pour l'adduit pipéridine-dibenzofulvène). Le peptide est ensuite détaché de la résine par traitement avec 15 mL d'un mélange de dichorométhane ($CH_2Cl_2$) et d'acide trifluoroacétique (TFA) (v/v = 99/1) (2 x 3 minutes). Le clivage est réalisé rapidement, et la solution est introduite dans 15 mL d'une solution méthanol/pyridine (v/v = 8/2). Après concentration, le résidu est précipité plusieurs fois dans du diéthylether glacé pour obtenir une poudre blanche. Le précurseur linéaire est ensuite mis à réagir dans du $CH_2Cl_2$ (0,5 mM) avec du PyBOP (3 équivalents) et du DIEA (4 équivalents). La formation du peptide cyclique est suivie par analyse HPLC, et la réaction est terminée au bout de 10 minutes. Le dichlorométhane ($CH_2Cl_2$) est ensuite évaporé. Le résidu huileux est précipité avec un mélange $CH_2Cl_2/Et_2O$ pour obtenir un peptide cyclique sous forme de poudre. Les chaînes sont ensuite déprotégées par traitement avec une solution de 1,4 g de dithiothréitol (DTT) dans un mélange TFA/TIS (TrIsopropylSilane)/$H_2O$ (v/v/v = 95/2,5/2,5) (concentration en peptide = 10 mM). Après 2 heures d'agitation, la solution est évaporée sous pression réduite pour donner une huile jaune qui est précipitée plusieurs fois avec du diéthylether glacé. Le résidu solide obtenu est ensuite dissous dans un mélange eau/acétonitrile, puis passé sur un filtre de polytétrafluoroéthylène (PTFE) dont le diamètre des pores est de 0,45 $\mu$m, puis purifié par chromatographie HPLC en phase inverse (gradient de 5 à 45% de B en 30 minutes), pour donner un composé **$P^1$** sous forme de poudre blanche (52 mg, 19% de rendement), ou un composé **$P^2$** sous forme de poudre blanche (144 mg, 53% de rendement).

**Composé $P^1$ :**

Analyse HPLC, pureté : 96%, $t_R$ = 15,7 min (gradient 5 à 60% de B en 30 minutes).
MS : calculé pour $C_{43}H_{62}N_{14}O_{12}S_2$, $[M+H]^+$ = 1031,41, exp = $[M+H^+]^+$ = 1031,45.

**Composé $P^2$ :**

Analyse HPLC, pureté : 98%, $t_R$ = 23,6 min (gradient 5 à 45% de B en 30 minutes).
MS : calculé pour $C_{45}H_{59}N_{11}O_{16}S_2$, $[M+H^+]^+$ = 1074,36, exp = $[M+H^+]^+$ = 1074,65.

**Synthèses de composés cyclodécapeptides $P^3$ et $P^3$-TRITC répondant à la formule (I) de l'invention (les acides aminés $Cys_2$ et $Cys_7$ étant liés par une liaison covalente) :**

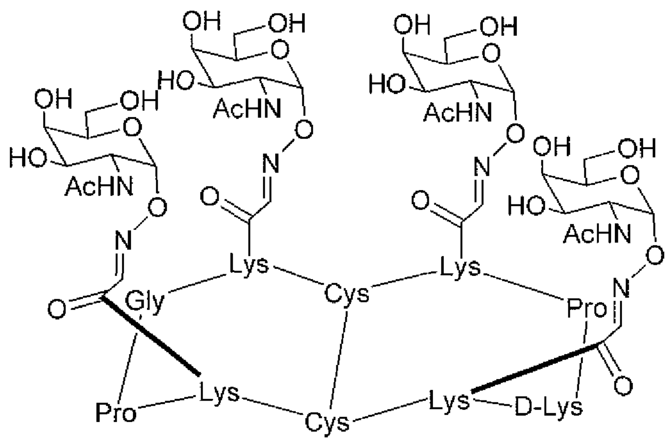

**$P^3$**

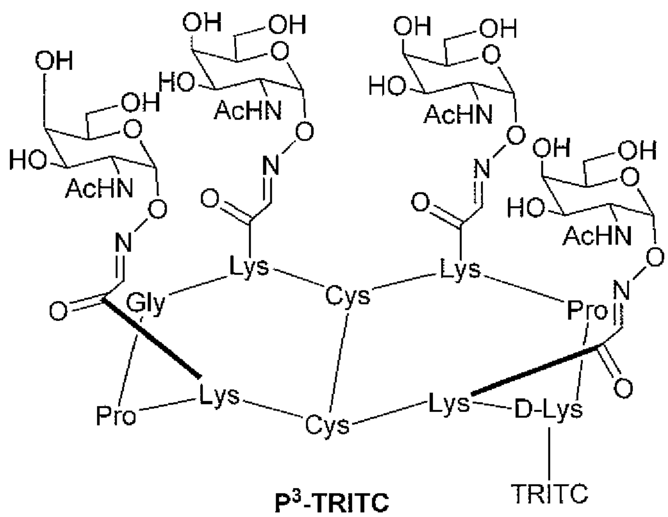

**$P^3$-TRITC**

Les composés cyclodécapeptides **$P^3$** et **$P^3$-TRITC** répondant à la formule (I) de l'invention sont synthétisés selon le schéma 1 ci-dessous. Les blocs oxyamines Fmoc-Lys[BocSer(tBu)]-OH et O-α-D-galactopyranosyl (αGaLNAcONH$_2$, composé **6**) sont synthétisés comme décrit dans la littérature (Renaudet et al., Org. Biomol. Chem., 2006, 4: 2628-2636; S. Fouillard et al., J. ORG. Chem., 2008, 73: 983-991).

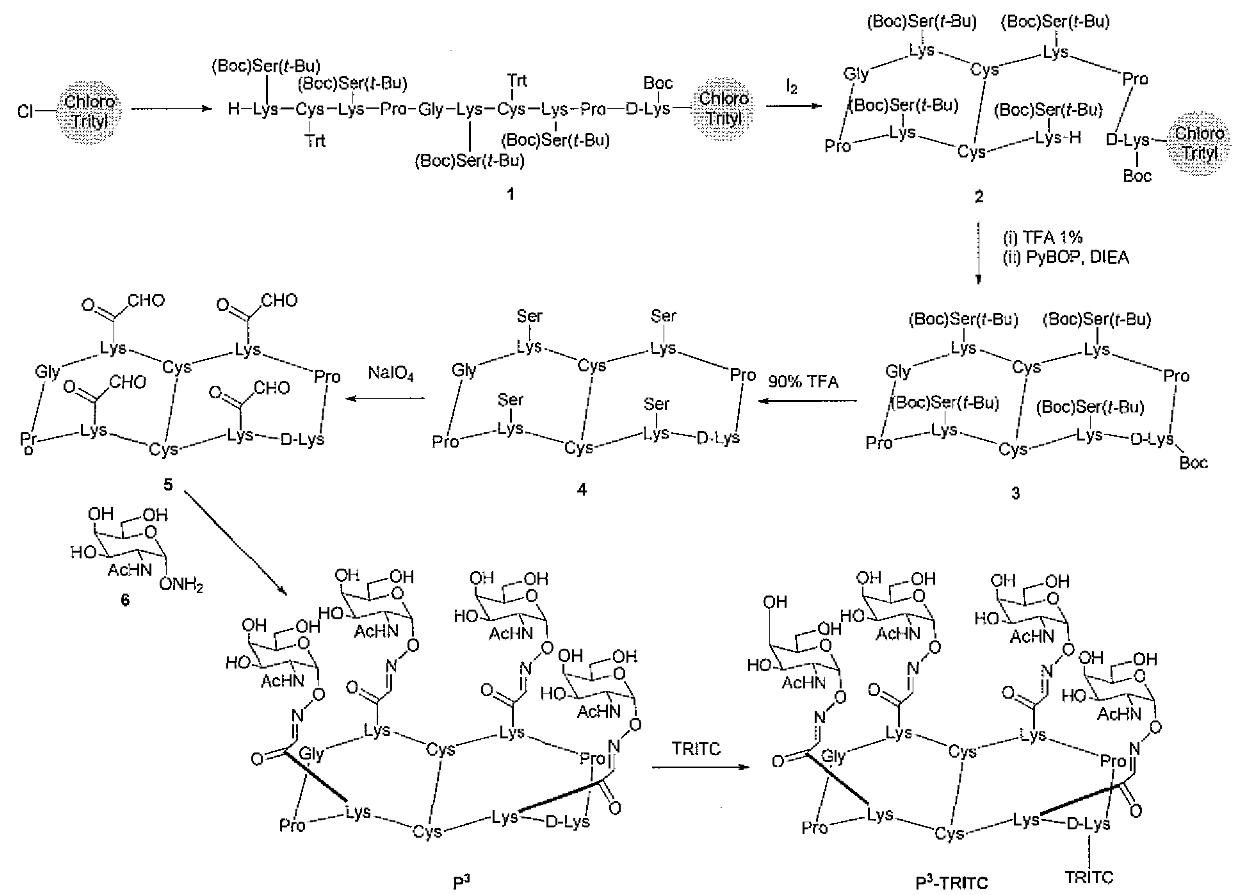


Schéma 1

**Synthèse du composé 4 :**

Le précurseur linéaire **1** porteur de groupements protecteurs est préparé par synthèse peptidique en phase solide sur une résine chlorure de chloro-2-trityle (substitution 0,4 mmol/g, 0,507 g, 0,202 mmol) par chimie Fmoc. La résine est gonflée avec du dichlorométhane ($CH_2Cl_2$) (10 mL, 1 x 10 min) et du DMF (10 mL, 1 x 10 min). Les couplages sont réalisés par mélange des acides aminés N-α-Fmoc-protégés ou Fmoc-Lys[BocSer(tBu)]-OH (2,5 équivalents, 0,5 mmol), avec du benzotriazol-1-yl-oxytripyrrolidinophosphonium (PyBOP) (2,5 équivalents, 0,5 mmol) et du N,N-diisopropyléthylamine (DIEA) (pH ≈ 8-9) dans du DMF (10 mL), pendant 30 minutes. Après lavage avec du DMF (10 mL, 4 x 1 min) et du $CH_2Cl_2$ (10 mL, 2 x 1 min), la déprotection des groupements N-α-Fmoc est réalisée par traitement avec un mélange DMF/pipéridine (v/v = 4/1, 10 mL, 3 x 10 min). Après un dernier lavage au DMF (10 mL, 6 x 1 min), la fin de la réaction de déprotection est contrôlée par spectrométrie UV-visible ($\varepsilon^{300\ nm}$ = 7800 L.mol-1.cm-1 pour l'adduit pipéridine-dibenzofulvène). Après la dernière réaction de couplage, la résine fonctionnalisée 1 est obtenue (0,13 mmol, 64% de rendement).

La résine fonctionnalisée **1** (0,13 mmol) est ensuite gonflée avec du dichlorométhane ($CH_2Cl_2$) (10 mL, 1 x 10 min) et du DMF (10 mL, 1 x 10 min). De l'iode (0,660 g, 2,60 mmol) et du DMF (10 mL) sont ajoutés. Le mélange réactionnel est agité à température ambiante pendant 1,5 heure. Après filtration, la résine est lavée avec du DMF (10 mL, 6 x 5 min), un mélange DMF/eau (v/v = 1/1) (10 mL, 2 x 5 min), du DMF (10 mL, 1 x 5 min) et du $CH_2Cl_2$ (10 mL, 3 x 5 min).

Le peptide est ensuite détaché de la résine par traitement avec un mélange $CH_2Cl_2$/TFA (v/v = 99/1, 10 mL, 10 x 2 min). Le filtrat est ensuite récupéré, et du N,N-diisopropyléthylamine (DIEA) (1 mL) est ajouté pour éviter la déprotection pendant l'étape d'évaporation. Après concentration, le résidu est précipité dans du diéthyléther. Le précurseur linéaire est ensuite mis à réagir dans du DMF (~ 0.5 mM) avec du PyBOP (0,074 g, 0,14 mmol) et du DIEA (0,08 mL, 0,39 mmol), pendant 2 heures. Le DMF est évaporé sous pression réduite. Le résidu huileux est précipité avec un mélange de $CH_2Cl_2/Et_2O$, pour donner un peptide cyclique **3** sous forme de poudre. Les chaînes sont ensuite déprotégées par traitement avec un mélange TFA/$H_2O$ (v/v = 90/10, 20 mL). Après 2 heures d'agitation, la solution est évaporée pour donner une huile jaune qui est précipitée avec du diéthyléther, pour donner un peptide **4** déprotégé sous forme de poudre blanche (0,097 g, 0,067 mmol, 33% de rendement).

Analyse HPLC, pureté : 83%, $t_R$ = 6,13 min (gradient linéaire A/B : 95/5 à 60/40, en 15 minutes).
MS : calculé pour $C_{60}H_{105}N_{19}O_{18}S_2$, $[M+H^+]^+$ = 1444,74, exp: $[M+H^+]^+$ = 1444,58, $[M+2H^+]^{2+}$ = 722,92, $[M+3H^+]^{3+}$ = 482,33.

**Synthèse du composé 5 :**

Du périodate de sodium (0,380 g, 1,77 mmol) est ajouté à une solution du composé **4** (0,064 g, 0,044 mmol) dans de l'eau (8 mL). Après 15 minutes, le mélange réactionnel est injecté dans une colonne RP-HPLC ($t_R$ = 14 min, gradient linéaire A/B : 95/5 à 60/40, en 15 minutes), pour donner un composé 5 sous forme de poudre blanche (0,009 g, 0,0068 mmol, 15% de rendement) après lyophilisation.
MS : calculé pour $C_{56}H_{85}N_{15}O_{18}S_2$, $[M+H^+]^+$ = 1320,57, exp = $[M+H^+]^+$ = 1320,5.

**Synthèse du composé P³ :**

De l'O-α-D-galactopyranosyl oxyamine (composé **6)** est ajoutée (0,045 g, 0,192 mmol) à une solution du composé **5** (0,025 g, 0,019 mmol), dans un mélange $AcOH/H_2O$ (4 mL, v/v = 1/9). Le mélange réactionnel est agité à temperature ambiante pendant 1 heure. Le mélange est ensuite injecté dans une colonne RP-HPLC ($t_R$ = 14-16 min, gradient linéaire A/B : 95/5 à 60/40, en 15 minutes), pour donner un composé **P³** sous forme de poudre blanche (0,021 g, 0,0096 mmol, 50% de rendement) après lyophilisation.

Analyse HPLC, pureté : 95%, $t_R$ = 7,0 min (gradient linéaire A/B : 95/5 à 60/40, en 15 minutes).
MS : calculé pour $C_{88}H_{141}N_{23}O_{38}S_2$, $[M+H^+]^+$ = 2192,92, exp = $[M+H^+]^+$ = 2193,5.

**Synthèse du composé P³-TRITC** :

Un marqueur, le TétraMéthylRhodamineIsoThioCyanate (TRITC) est ajouté (0,003 g, 0,0067 mmol) avec quelques gouttes de DIEA (pH ≈ 8-9) à une solution d'un composé **P³** (0,012 g, 0,0055 mmol) dans du DMF (2 mL). Le mélange réactionnel est ensuite agité à température ambiante pendant 2 heures, puis injecté dans une colonne RP-HPLC ($t_R$ = 19 min, gradient linéaire A/B : 95/5 à 60/40, en 15 minutes) pour donner un composé **P³-TRITC** sous forme de poudre blanche (0,0012 g, 0,00046 mmol, 8% de rendement) après lyophilisation.
MS : calculé pour $C_{113}H_{163}N_{26}O_{41}S_3^+$, $[M]^+$ = 2637,1, exp = $[M]^+$ = 2636,6, $[M^++H^+]^{2+}$ = 1318,9.

**C - <u>Caractérisation des complexes du cuivre Cu(I) formés avec les peptides P¹ et P² Mode opératoire</u> :**

Les fonctions thiols -SH des acides aminés cystéines étant susceptibles de s'oxyder à l'air, toutes les solutions ont été préparées dans une boîte à gants sous atmosphère d'argon. Des solutions de ligands ont ensuite été préparées, avant chaque expérience, en utilisant une eau désoxygénée et purifiée par un système Millipore Milli-Q® contenant 20 mM d'une solution de tampon phosphate (pH = 7,4) et de l'acétonitrile (vlv : 9/1).

La concentration finale de la solution a été déterminée par mesure de la concentration des fonctions thiols libres, en suivant la procédure d'Ellman décrite dans la littérature (P. W. Riddles et al., Methods Enzymol., 1983, 91, pp. 49-60). Cette méthode utilise de l'acide 5,5'-dithiobis-2-nitrobenzoïque (DNTB) comme indicateur, chaque groupement thiol libre présent dans le ligand conduisant à 1 équivalent de $TNB^{2-}$ ($\varepsilon^{412\ nm}$ ($TNB^{2-}$) = 14 150 $M^{-1}.cm^{-1}$, $\varepsilon^{412\ nm}$ étant le coefficient d'extinction molaire du $TNB^{2-}$ à 412 nm). Les concentrations des solutions de ligands sont comprises entre 30 et 100 μM.

Les solutions de cuivre Cu(I) ont été préparées en dissolvant une quantité appropriée de $Cu(CH_3CN)_4PF_6$ dans de l'acétonitrile désoxygénée. La concentration finale est déterminée par ajout d'un excès de disulfonate bathocuproïne de sodium ($Na_2BCS$) et par mesure de l'absorbance du $Cu(BCS)_2^{3-}$ ($\lambda_{max}$ = 483 nm, ε = 13 300 $M^{-1}.cm^{-1}$).

Pour les mesures de constantes d'affinité, le complexe est préparé en ajoutant à la solution de ligand une solution d'acétonitrile ($CH_3CN$) contenant 0,8-0,9 équivalent de cuivre Cu(I), dans une solution tampon phosphate à 20 mM (pH = 7,4) et d'acétonitrile ($CH_3CN$) (v/v : 9/1).

La formation du complexe est ensuite réalisée par agitation du mélange pendant 10 minutes, sous argon. Des aliquotes d'une solution de disulfonate bathocuproïne (BCS) dans la même solution tampon sont ensuite ajoutés au complexe ligand-cuivre. Les spectres UV-visible sont enregistrés, et la stabilité de l'absorbance est contrôlée avant l'addition des autres aliquotes.

**1- Spectroscopie UV-visible**

La formation des complexes du Cu(I) a été suivie par spectroscopie UV-visible.

La Figure 1 donne un exemple de dosage UV du composé **$P^1$** par du $Cu(CH_3CN)PF_6$ (Cu(I)) en tampon phosphate à 20 mM, à un pH de 7,4 et à 298 K.

La bande de transfert de charge thiolate → Cu(I) apparaît clairement autour de 260 nm. Cette bande croît jusqu'à 1 équivalent pour les deux peptides **$P^1$** et **$P^2$**. Les complexes du Cu(I) obtenus ont donc une stoechiométrie globale de 1:1 (Cu:L) pour ces ligands (L) comportant deux cystéines.

**2- Spectrométrie de masse**

La stoechiométrie du complexe est aussi démontrée par spectrométrie de masse en mode d'ionisation électrospray, les spectres de masse ayant été enregistrés avec un spectromètre de type LXQ THERMO SCIENTIFIC. Le complexe Cu(**$P^1$**) est clairement détecté sur les spectres du composé **$P^1$** en présence de 1 équivalent de Cu(I) (cf. Figure 2).

**3- Constantes d'affinité**

Pour les mesures de constantes d'affinité, le complexe est préparé en ajoutant à la solution de ligand une solution d'acétonitrile ($CH_3CN$) contenant 0,8-0,9 équivalents de cuivre Cu(I), dans une solution tampon phosphate à 20 mM (pH = 7,4) et d'acétonitrile ($CH_3CN$) (v/v : 9/1).

L'affinité des composés de l'invention pour le Cu(I) est une donnée importante puisqu'elle permet de quantifier la capacité des composés de l'invention à complexer cet ion.

Les constantes d'affinité ont été mesurées grâce à un compétiteur connu ayant une forte affinité pour le Cu(I), le disulfonate bathocuproïne (BCS), qui forme des complexes avec le cuivre Cu(I) de stabilité connue selon la réaction ci-dessous (Z. Xiao et al., J. Am. Chem. Soc., 2004, 126 : 3081-3090; P. Rousselot-Paillet et al., Inorg. Chem., 2006, 45: 5510-5520):

$$\mathrm{Cu(I)} + 2\ \mathrm{BCS} = \mathrm{Cu(BCS)_2} \qquad K = \frac{[\mathrm{Cu(BCS)_2}]}{[\mathrm{Cu}][\mathrm{BCS}]^2} = 10^{19,8} \text{ à } 298\ \mathrm{K}$$

Ces expériences de compétition ont permis de quantifier l'affinité des composés **$P^1$** et **$P^2$** de l'invention pour le cuivre Cu(I) : les constantes apparentes de complexation du cuivre Cu(I), dans une solution tampon phosphate à 20 mM de pH 7,4, à une température de 298 K, telles que définies ci-dessous, sont données au Tableau II.

$$K_{app} = \frac{[Cu]_{complexé}}{[Cu]_{libre}[L]_{libre}}$$

**Tableau II :**

| | **$P^C$** | Composé **$P^1$** | Composé **$P^2$** |
|---|---|---|---|
| $\log K_{app}$ | 16,5 | 16,7 | 15,5 |

Il apparaît clairement que les peptides de l'invention dans lesquels les fonctions thiols des acides aminés cystéines sont libres ont de fortes affinités pour le Cu(I). Par ailleurs, les affinités de **$P^1$** et **$P^2$** sont comparables à celles obtenues avec le cyclopeptide **$P^c$** (référence) modélisant la boucle chaperonne à cuivre de la levure Atxl (P. Rousselot-Paillet et al., Inorg. Chem., 2006, 45 : 5510-5520). Ces résultats démontrent la capacité des composés de l'invention à complexer le cuivre Cu(I) en excès dans un milieu intracellulaire.

**D/ Caractérisation des complexes entre les peptides $P^1$ et $P^2$ et d'autres ions métalliques Mode opératoire :**

Les procédures sont celles décrites dans l'article Rousselot-Paillet et al., Inorg. Chem., 2006, 4 : 2628-2635.

La complexation du mercure Hg(II) par les composés **$P^1$** et **$P^2$** est très efficace (constantes d'affinité élevées), et peut donc présenter un intérêt pour la détoxication de ce métal toxique.

La complexation du zinc Zn(II) a également été étudiée car cet ion métallique non toxique est présent *in vivo,* dans les cellules hépatiques visées. Les composés ont beaucoup moins d'affinité pour le zinc Zn(II) que pour le cuivre

Cu(I) et le mercure Hg(II). Cette sélectivité est primordiale car elle permet de détoxiquer le métal visé (cuivre Cu(I) ou mercure Hg(II)) sans complexer le zinc. Ce paramètre est exprimé par la sélectivité Sél. M/M' entre deux métaux M et M'.

Le Tableau III ci-dessous rassemble les constantes apparentes obtenues à pH = 7,4, avec les composés cyclodécapeptides de l'invention, et leur sélectivité pour les ions visés par rapport au zinc Zn(II).

**Tableau III**

| **$logK^{app}$** | **$P^C$** | Composé **$P^1$** | Composé **$P^2$** |
|---|---|---|---|
| Cu(I) | 16,5 | 16,7 | 15,5 |
| Zn(II) | 6,8 | 6,6 | 5,9 |
| Hg(II) | > 18,6 | > 18,7 | > 17.5 |
| Sél. Cu/Zn | 9,7 | 10,1 | 9,6 |
| Sél. Hg/Zn | > 11,8 | > 12,1 | 11,6 |

Les composés **$P^1$** et **$P^2$** de l'invention présentent de bonnes affinités et de bonnes sélectivités par rapport au zinc Zn(II) présent dans les cellules, ce qui les rend très prometteurs pour la complexation sélective du cuivre ayant un degré d'oxydation +I, degré d'oxydation favorisé dans le milieu intracellulaire et qui peut donc être ciblé dans les maladies de type Wilson. Ces composés sont également des candidats pour la complexation sélective du mercure Hg(II) lors d'intoxications par ce métal.

**E/ Caractérisation des complexes du cuivre Cu(I) formé avec le peptide $P^3$**

Le BCS a été utilisé pour déterminer la concentration en cuivre Cu(I) non complexé par le composé **$P^3$**. Les résultats sont représentés à la Figure 3. Lorsque le composé **$P^3$** est seul, il ne complexe pas le cuivre (100% de Cu(I) est complexé par le BCS), puisque les fonctions thiols du composé **$P^3$** sont masquées par les ponts disulfure (S-S) de la molécule. Par contre, en présence d'un réducteur (GSH 1 mM comme dans les cellules), capable de réduire les ponts S-S pour régénérer les fonctions thiols libres, le composé **$P^3$** évolue vers un complexant efficace du cuivre (les deux cystéines $Cys_2$ et $Cys_7$ étant alors libres comme dans les composés **$P^1$** ou **$P^2$**), puisque la quantité de cuivre détectée par le BCS chute à 40%. Ce pourcentage correspond à une constante de stabilité apparente intermédiaire entre les peptides **$P^1$** et **$P^2$** : $logK^{app}$ (**$P^3$** réduit) = 16.

Ces résultats montrent que le composé **$P^3$** complexe le cuivre Cu(I) en milieu réducteur, avec une affinité similaire aux composés **$P^1$** et **$P^2$**, les composés de l'invention dans lesquels les acides aminés cystéines $Cys_2$ et $Cys_7$ sont liés par une liaison covalente $Cys_2$-$Cys_7$ ne devenant des agents chélatants de métaux uniquement dans les cellules ciblées, et ne provoquant donc pas d'effets secondaires liés à la complexation indésirée de métaux dans d'autres endroits de l'organisme.

**F/ Résultats biologiques sur cellules hépatiques**

**1- Entrée du composé $P^3$-TRITC dans les cellules hépatiques**

**Mode opératoire :**

Les cellules ($10^5$-$10^6$/mL) sont déposées sur des lamelles au fond de puits de culture et immergées dans le milieu de culture approprié. Après un temps d'incubation variable en présence du composé **$P^3$-TRITC,** chaque lamelle est lavée, fixée par une solution de formaldéhyde à 10% (Sigma) et montée sur une lame d'observation en présence de liquide de montage (Sigma). Chaque lame est ensuite observée au microscope à fluorescence pour localiser le marqueur TRITC dans les cellules. Une trentaine de champs sont observés sur chaque lamelle pour obtenir un résultat statistique significatif. L'expérience est répétée sur des cellules provenant de différents lots.

**Résultats :**

L'entrée du composé **$P^3$-TRITC** (0,2 μM) dans des cellules hépatiques de type HepG2, WIF-B9 (C. Decaens et al., 1996, J. Cell Sci., 109 (Pt 6) : 1623-1635) et Can10 (X. Peng et al., 2006, Cell Tissue Res., 323 : 233-243) a été étudiée par microscopie à fluorescence en suivant l'émission dans le rouge du marqueur TRITC (cf. Figure 4). Des cinétiques ont été réalisées à deux concentrations (0,2 et 2 μM), pour évaluer le temps d'entrée de la molécule dans

ces différentes cellules.

Les hépatocytes de la lignée HepG2 incorporent le composé **$P^3$-TRITC** dès 2 heures d'incubation. Avec le temps, les cellules s'enrichissent en composé **$P^3$-TRITC.**

L'étude a été poursuivie avec les hépatocytes des lignées Can10 (cf. Figure 5) et WIF-B9, qui ont la particularité de former des canalicules biliaires au bout d'une dizaine de jours de culture. Cette caractéristique permet de suivre le devenir du composé **$P^3$-TRITC** dans une cellule polarisée, plus proche de la physiologie que la lignée HepG2 qui ne se polarise pas. Par rapport à la situation d'un hépatocyte dans le foie, le milieu de culture figure le plasma sanguin et le canalicule biliaire figure le lieu d'excrétion naturel du cuivre.

Les cellules Can10 incorporent le composé **$P^3$-TRITC** dès 2 heures d'incubation. Au bout de 24 heures d'incubation, certains canalicules sont fluorescents, ce qui démontre que le composé **$P^3$-TRITC** a traversé les cellules. Après 48 heures, tous les canalicules sont fluorescents.

Les résultats obtenus avec les cellules de la lignée WIF-B9 sont similaires.

Ces résultats démontrent que le composé **$P^3$-TRITC** est capable de rentrer dans différents types de cellules hépatiques en seulement quelques heures.

2- Complexation du cuivre **Cu(I)** dans les cellules hépatiques

Mode **opératoire** :

Les cellules ($10^5$-$10^6$/mL) sont déposées sur des lamelles au fond de puits de culture et immergées dans le milieu de culture approprié. Après une incubation de 1 à 5 heures en présence de 1 μM de cuivre Cu(I), et éventuellement en présence de 10 μM de composé **$P^3$**, chaque lamelle est lavée, puis fixée et les cellules perméabilisées par une solution de méthanol pur à -20°C pendant 4 minutes. Après lavage, les lamelles sont exposées à un milieu contenant l'anticorps primaire anti-ATP7B (Hernandez et al., Gastoenterology, 2008, 134, 1215-1223) et, en cas de double marquage, à un milieu contenant un anticorps anti-ZO-1 (ZO-1 étant une protéine constituée de jonctions serrées associant les hépatocytes et délimitant la membrane apicale, ZO-1 étant un marqueur des canalicules). Les lamelles sont ensuite exposées à un milieu contenant un anticorps secondaire, Alexa Fluor 546 goat anti-rat IgG (H + L) (Invitrogen), fluorescent dans le vert pour la protéine ATP7B et dans le rouge pour l'anticorps ZO-1, puis montées sur une lame d'observation en présence de liquide de montage (Sigma), cet anticorps secondaire permettant de visualiser la protéine ZO-1 et d'en signaler la position dans les cellules. Chaque lame est ensuite observée au microscope à fluorescence pour localiser la protéine ATP7B, et éventuellement ZO-1, dans les cellules. Une trentaine de champs sont observés sur chaque lamelle pour obtenir un résultat statistique significatif. L'expérience est répétée sur des cellules provenant de différents lots.

**Résultats :**

Il a été montré que la position de la protéine ATP7B (protéine de Wilson) membranaire dépendait de la concentration en cuivre Cu(I) intracellulaire dans les hépatocytes, comme par exemple le WIF-B9. Cette protéine peut donc être utilisée comme indicateur de l'augmentation de la concentration intracellulaire de cuivre Cu(I). Pour cela, la position de la protéine ATP7B dans la cellule est repérée par un marquage avec un anticorps primaire anti-Wilson, lui-même détecté par un anticorps secondaire fluorescent dans le vert. En conditions basales, la protéine ATP7B se situe dans la région de l'appareil de Golgi, alors qu'en excès de cuivre Cu(I) elle se déplace vers la membrane apicale, c'est-à-dire vers la membrane qui entoure les canalicules, pour excréter l'excès de cuivre Cu(I) (Y. Guo et al., Am. J. Physiol. Gastrointest. Liver. Physiol., 2005, 289 : G904-G916).

La localisation de la protéine ATP7B dans les cellules WIF-B9 est représentée à la Figure 6. Dans les conditions basales (image A), la protéine ATP7B est proche de l'appareil de Golgi, entre la canalicule biliaire (Cb) et le noyau (N). Après 2 heures d'incubation en présence de 1 μM de cuivre Cu(I), la protéine ATP7B se rapproche de la membrane apicale, jusqu'à entourer les canalicules.

Les expériences réalisées sur les cellules WIF-B9 ont également démontré qu'il était possible de voir le déplacement de la protéine ATP7B entre les conditions basales (cuivre ~ 0.01 μM) et un excès de cuivre Cu(I) (1 μM). Ce déplacement peut donc être utilisé comme une sonde de la concentration intracellulaire du cuivre Cu(I).

Pour éprouver la capacité du composé **$P^3$** à diminuer la concentration intracellulaire du cuivre Cu(I), les cellules incubées en excès de cuivre Cu(I) sont mises en présence du composé **$P^3$** pendant au moins 2 heures. L'incubation avec le composé **$P^3$** inhibe le déplacement de la protéine ATP7B vers la membrane apicale, ce qui démontre l'absence d'augmentation de la concentration intracellulaire en cuivre Cu(I). Le composé **$P^3$** s'avère donc être un agent chélatant du cuivre Cu(I) *in cellulo.*

La position de la protéine ATP7B en présence de 1 μM de cuivre Cu(I), en absence (images A) ou en présence (images B) de 10 μM de composé **$P^3$** est également représentée à la Figure 7 (à gauche : image par contraste de phase

(Nomarski) ; au centre : fluorescence de la protéine ATP7B (visualisé au moyen de l'anticorps Alexa Fluor 488) ; à droite : fluorescence de ZO-1 (protéine des jonctions serrées ou *zonula occludens* visualisée au moyen de l'anticorps Alexa Fluor 546 ; les jonctions serrées sont situées à l'apex des hépatocytes et contiennent de nombreuse protéines, dont ZO-1, et elles assurent l'association des cellules entre elles, et donc l'étanchéité entre l'espace interne des canicules et le milieu intercellulaire).

## Revendications

**1.** Composé cyclodécapeptide répondant à la formule (I) suivante :

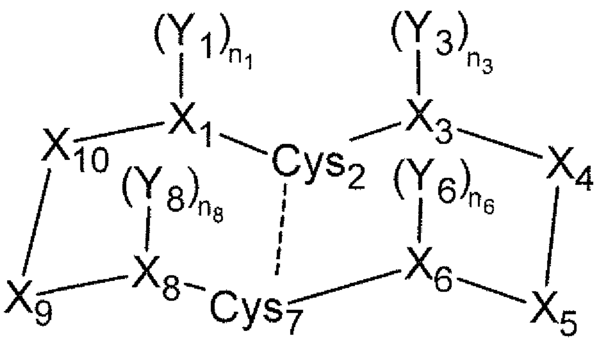

(I)

dans lequel :

- ✓ les acides aminés cystéines $Cys_2$ et $Cys_7$ peuvent être liés ou non par une liaison covalente $Cys_2$-$Cys_7$ via leurs atomes de soufre,
- ✓ $X_1$, $X_3$, $X_4$, $X_5$, $X_6$, $X_8$, $X_9$, $X_{10}$, identiques ou différents, sont des acides aminés présents sous leur forme dextrogyre (D) ou lévogyre (L),
- ✓ $n_1$, $n_3$, $n_6$, $n_8$, identiques ou différents, sont égaux à 0 ou 1,
- ✓ $Y_1$, $Y_3$, $Y_6$, Yg, identiques ou différents, représentent des groupements-C(O)CHNL, -C(O)EL ou -NHEL, dans lesquels L est un ligand biologique, et E est un bras espaceur sélectionné parmi les polyols et les chaînes alkyles éventuellement substituées ayant 1 à 12 atomes de carbone,
- ✓ en option, au moins un des acides aminés $X_4$, $X_5$, $X_9$, $X_{10}$, et/ou au moins un des groupements $Y_1$, $Y_3$, $Y_6$, $Y_8$, peut être substitué par un groupement sélectionné parmi : -CO-marqueur, -NH-marqueur, -C(S)NH-marqueur, -$SO_2$-marqueur, =CH-marqueur, -E'-marqueur, où E' est un bras espaceur sélectionné parmi le phényle, le triazole, l'oxadiazole, l'oxazole, l'imidazole, le thiadiazole, le pyrrole, le tétrazole, le furane, le thiophène, le pyrazole, la pyrazoline, la pyrazidine, le thiazole, l'isothiazole, la pyridine, la pyrimidine, la pipéridine, le pyranne, la pyrazine, la pyridazine et leurs dérivés, et

étant entendu que les liaisons $X_4$-$X_5$ et $X_9$-$X_{10}$, identiques ou différentes, sont choisies parmi les liaisons (D)Pro-(L)X ou (L)Pro-(D)X', dans lesquelles X et X' sont des acides aminés
pour leur utilisation en tant que médicaments.

**2.** Composé de formule (I) pour son utilisation selon la revendication 1, dans lequel l'un au moins des acides aminés $X_1$, $X_3$, $X_6$, $X_8$ est une lysine.

**3.** Composé de formule (I) pour son utilisation selon l'une des revendications 1 ou 2, dans lequel X et X' sont choisi parmi la glycine, la lysine, le glutamate ou l'aspartate.

**4.** Composé de formule (I) pour son utilisation selon l'une des revendications 1 à 3, dans lequel au moins un des groupements $Y_1$, $Y_3$, $Y_6$, $Y_8$ représente un groupement-C(O)CHNL.

**5.** Composé de formule (I) pour son utilisation selon l'une des revendications 1 à 4, dans lequel le ligand L est un ligand biologique de cellules hépatiques ou neuronales, sélectionné parmi les oses tels que le glucose, le galactose et le N-acétylgalactosamine.

6. Composé de formule (I) pour son utilisation selon l'une des revendications 1 à 5, dans lequel le marqueur est un fluorophore choisi parmi la rhodamine, la fluorescéïne, la pyronine, la coumarine, la benzophénone, l'anthrone, la fluorénone, la pyridine, la quinoléine, l'acridine, le naphtalène, l'anthracène, le naphtacène, le pentacène, le xanthène et leurs dérivés.

7. Composé de formule (I) pour son utilisation selon l'une des revendications 1 à 6, dans lequel au moins un des acides aminés $X_4$, $X_5$, $X_9$, $X_{10}$, et/ou au moins un des groupements $Y_1$, $Y_3$, $Y_6$, $Y_8$, est substitué par un groupement sélectionné parmi : -CO-marqueur, -NH-marqueur, -C(S)NH-marqueur, -$SO_2$-marqueur, =CH-marqueur, -E'-marqueur.

8. Composé de formule (I) pour son utilisation selon l'une des revendications 1 à 7, dans lequel au moins un des acides aminés $X_1$, $X_3$, $X_6$, $X_8$ est une lysine portant un groupement $Y_1$, $Y_3$, $Y_6$, $Y_8$.

9. Composé de formule (I) pour son utilisation selon l'une des revendications 1 à 7, dans lequel $n_1$, $n_3$, $n_6$, $n_8$ = 0, ledit composé répondant à la formule (Ia) suivante :

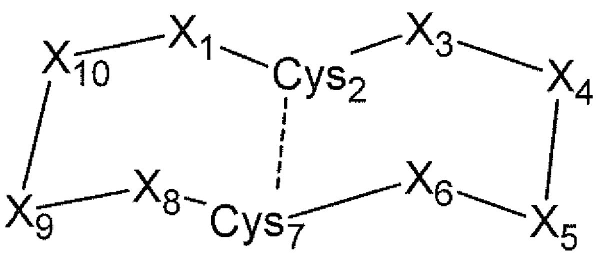

(Ia)

10. Utilisation des composés de formule (I) selon l'une quelconque des revendications 1 à 9, pour la préparation d'un médicament destiné au diagnostic, à la prévention et/ou au traitement de maladies neurodégénératives.

11. Utilisation selon la revendication 10, pour le traitement de la maladie de Wilson ou pour le traitement de la maladie d'Alzheimer.

12. Utilisation des composés de formule (I) selon l'une quelconque des revendications 1 à 9, pour la préparation d'un médicament destiné au diagnostic, à la prévention et/ou au traitement d'intoxications par des ions métalliques tels que les ions argent, cadmium, cobalt, cuivre, mercure, nickel, or, plomb et zinc.

13. Utilisation selon la revendication 12, pour la préparation d'un médicament destiné au traitement d'intoxications par des ions cuivre ou par des ions mercure.

14. Composition pharmaceutique **caractérisée en ce qu'**elle comprend en tant que principe actif au moins un composé de formule (I) selon l'une quelconque des revendications 1 à 9, et au moins un véhicule pharmaceutiquement acceptable.

## Patentansprüche

1. Cyclodecapeptidverbindung nach folgender Formel (I):

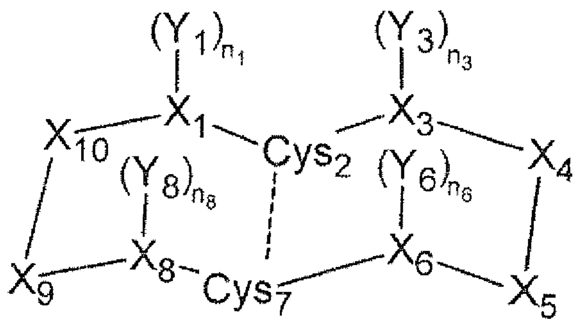

(I)

wobei:

- die Cystein-Aminosäuren $Cys_2$ und $Cys_7$ anhand einer kovalenten $Cys_2$-$Cys_7$-Bindung über ihre Schwefelatome gebunden sein können oder nicht,
- $X_1$, $X_3$, $X_4$, $X_5$, $X_6$, $X_8$, $X_9$, $X_{10}$, identisch oder unterschiedlich, Aminosäuren sind, die in ihrer rechtsdrehenden (D) oder linksdrehenden Form (L) vorkommen,
- $n_1$, $n_3$, $n_6$, $n_8$, identisch oder unterschiedlich, gleich 0 oder 1 sind,
- $Y_1$, $Y_3$, $Y_6$, $Y_8$, identisch oder unterschiedlich, -C(O)CHNL-, -C(O)EL- oder -NHEL-Gruppen darstellen, wobei L ein biologischer Ligand ist und E ein Spacerarm ist, der aus den Polyolen und den eventuell substituierten Alkylketten mit 1 bis 12 Kohlenstoffatomen ausgewählt ist,
- optional mindestens eine der Aminosäuren $X_4$, $X_5$, $X_9$, $X_{10}$ und/oder mindestens eine der Gruppen $Y_1$, $Y_3$, $Y_6$, $Y_8$ durch eine Gruppe substituierbar ist, die ausgewählt ist aus: -CO-Marker, -NH-Marker, -C(S)NH-Marker, -$SO_2$-Marker, =CH-Marker, -E'-Marker, wobei E' ein Spacerarm ist, der aus dem Phenyl, dem Thiazol, dem Oxadiazol, dem Oxazol, dem Imidazol, dem Thiadiazol, dem Pyrrol, dem Tetrazol, dem Furan, dem Thiophen, dem Pyrazol, dem Pyrazolin, dem Pyrazidin, dem Thiazol, dem Isothiazol, dem Pyridin, dem Pyrimidin, dem Piperidin, dem Pyrann, dem Pyrazin, dem Pyridazin und deren Derivaten ausgewählt ist, und

wobei es sich versteht, dass die Bindungen $X_4$-$X_5$ und $X_9$-$X_{10}$, identisch oder unterschiedlich, aus den Bindungen (D)Pro-(L)X oder (L)Pro-(D)X' ausgewählt sind, wobei X und X' Aminosäuren sind,
für ihre Verwendung als Arzneimittel.

**2.** Verbindung der Formel (I) für ihre Verwendung nach Anspruch 1, wobei mindestens eine der Aminosäuren $X_1$, $X_3$, $X_6$, $X_8$ ein Lysin ist.

**3.** Verbindung der Formel (I) für ihre Verwendung nach einem der Ansprüche 1 oder 2, wobei X und X' aus dem Glycin, dem Lysin, dem Glutamat oder dem Aspartat ausgewählt sind.

**4.** Verbindung der Formel (I) für ihre Verwendung nach einem der Ansprüche 1 bis 3, wobei mindestens eine der Gruppen $Y_1$, $Y_3$, $Y_6$, $Y_8$ eine -C(O)CHNL-Gruppe darstellt.

**5.** Verbindung der Formel (I) für ihre Verwendung nach einem der Ansprüche 1 bis 4, wobei der Ligand L ein biologischer Ligand von Leber- oder neuronalen Zellen ist, ausgewählt aus den Osen wie die Glucose, die Galactose und das N-acetylgalactosamin.

**6.** Verbindung der Formel (I) für ihre Verwendung nach einem der Ansprüche 1 bis 5, wobei der Marker ein Fluorophor ist, ausgewählt aus dem Rhodamin, dem Fluorescein, dem Pyronin, dem Coumarin, dem Benzophenon, dem Anthron, dem Fluorenon, dem Pyridin, dem Chinolin, dem Acridin, dem Naphtalen, dem Anthracen, dem Naphtacen, dem Pentacen, dem Xanthen und deren Derivaten.

**7.** Verbindung der Formel (I) für ihre Verwendung nach einem der Ansprüche 1 bis 6, wobei mindestens eine der Aminosäuren $X_4$, $X_5$, $X_9$, $X_{10}$ und/oder mindestens eine der Gruppen $Y_1$, $Y_3$, $Y_6$, $Y_8$ von einer Gruppe substituiert ist, die ausgewählt ist aus: -CO-Marker, -NH-Marker, -C(S)NH-Marker, -$SO_2$-Marker, =CH-Marker, -E'-Marker.

**8.** Verbindung der Formel (I) für ihre Verwendung nach einem der Ansprüche 1 bis 7, wobei mindestens eine der

Aminosäuren $X_1$, $X_3$, $X_6$, $X_8$ ein Lysin mit einer Gruppe $Y_1$, $Y_3$, $Y_6$, $Y_8$ ist.

**9.** Verbindung der Formel (I) für ihre Verwendung nach einem der Ansprüche 1 bis 7, wobei $n_1$, $n_3$, $n_6$, $n_8$ = 0, wobei die Verbindung der folgenden Formel (Ia) entspricht:

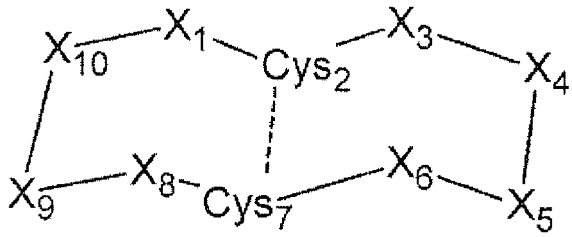

(Ia)

**10.** Verwendung der Verbindungen der Formel (I) nach einem der Ansprüche 1 bis 9 für die Herstellung eines Arzneimittels, das für die Diagnose, die Prävention und/oder die Behandlung von neurodegenerativen Erkrankungen bestimmt ist.

**11.** Verwendung nach Anspruch 10 für die Behandlung von Morbus Wilson oder für die Behandlung der Alzheimer-Krankheit.

**12.** Verwendung der Verbindungen der Formel (I) nach einem der Ansprüche 1 bis 9 für die Herstellung eines Arzneimittels, das für die Diagnose, die Prävention und/oder die Behandlung von Intoxikationen durch Metallionen wie Silber-, Kadmium-, Kobalt-, Kupfer-, Quecksilber-, Nickel-, Gold-, Blei- und Zinkionen bestimmt ist.

**13.** Verwendung nach Anspruch 12 für die Behandlung von Intoxikationen durch Kupferionen oder durch Quecksilberionen.

**14.** Pharmazeutische Zusammensetzung, **dadurch gekennzeichnet, dass** sie als Wirkstoff mindestens eine Verbindung der Formel (I) nach einem der Ansprüche 1 bis 9 und mindestens ein pharmazeutisch akzeptables Vehikel umfasst.

## Claims

**1.** A cyclodecapeptide compound corresponding to the following formula (I):

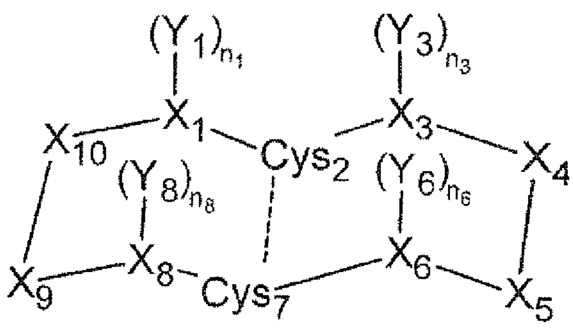

(I)

in which:

- the cysteine amino acids $Cys_2$ and $Cys_7$ may or may not be linked by a covalent bond $Cys_2$-$Cys_7$ via their sulfur atoms,
- $X_1$, $X_3$, $X_4$, $X_5$, $X_6$, $X_8$, $X_9$, $X_{10}$, which are identical or different, are amino acids present in their dextrorotatory

(D) or levorotatory form (L),
- $n_1$, $n_3$, $n_6$, $n_8$, which are identical or different, are equal to 0 or 1,
- $Y_1$, $Y_3$, $Y_6$, $Y_8$, which are identical or different, represent groups -C(O)CHNL, -C(O)EL or -NHEL, in which L is a biological ligand, and E is a spacer arm selected from polyols and optionally substituted alkyl chains having 1 to 12 carbon atoms,
- optionally, at least one of the amino acids $X_4$, $X_5$, $X_9$, $X_{10}$, and/or at least one of the groups $Y_1$, $Y_3$, $Y_6$, $Y_8$, may be substituted with a group selected from: -CO-marker, -NH-marker, -C(S)NH-marker, $-SO_2$-marker, =CH-marker, -E'-marker, where E' is a spacer arm selected from phenyl, triazole, oxadiazole, oxazole, imidazole, thiadiazole, pyrrole, tetrazole, furan, thiophene, pyrazole, pyrazoline, pyrazidine, thiazole, isothiazole, pyridine, pyrimidine, piperidine, pyran, pyrazine, pyridazine and derivatives thereof, and

it being understood that the bonds $X_4$-$X_5$ and $X_9$-$X_{10}$, which are identical or different, are chosen from the bonds (D)Pro-(L)X or (L)Pro-(D)X', in which X and X' are amino acids,
for use thereof as drugs.

2. The compound of formula (I) for use according to claim 1, wherein at least one of the amino acids $X_1$, $X_3$, $X_6$, $X_8$ is a lysine.

3. The compound of formula (I) for use according to one of claims 1 or 2, wherein X and X' are chosen from glycine, lysine, glutamate or aspartate.

4. The compound of formula (I) for use according to one of claims 1 to 3, wherein at least one of the groups $Y_1$, $Y_3$, $Y_6$, $Y_8$ represents a group -C(O)CHNL.

5. The compound of formula (I) for use according to one of claims 1 to 4, wherein the ligand L is a biological ligand for hepatic or neuronal cells, selected from monosaccharides such as glucose, galactose and N-acetylgalactosamine.

6. The compound of formula (I) for use according to one of claims 1 to 5, wherein the marker is a fluorophore chosen from rhodamine, fluorescein, pyronin, coumarin, benzophenone, anthrone, fluorenone, pyridine, quinoline, acridine, naphthalene, anthracene, naphthacene, pentacene, xanthene and derivatives thereof.

7. The compound of formula (I) for use according to one of claims 1 to 6, wherein at least one of the amino acids $X_4$, $X_5$, $X_9$. $X_{10}$, and/or at least one of the groups $Y_1$, $Y_3$, $Y_6$, $Y_8$, is substituted with a group selected from: -CO-marker, -NH-marker, -C(S)NH-marker, $-SO_2$-marker, =CH-marker, and -E'-marker.

8. The compound of formula (I) for use according to one of claims 1 to 7, wherein at least one of the amino acids $X_1$, $X_3$, $X_6$, $X_8$ is a lysine bearing a group $Y_1$, $Y_3$, $Y_6$, $Y_8$.

9. The compound of formula (I) for use according to one of claims 1 to 7, wherein $n_1$, $n_3$, $n_6$, $n_8$=0, said compound corresponding to the following formula (Ia):

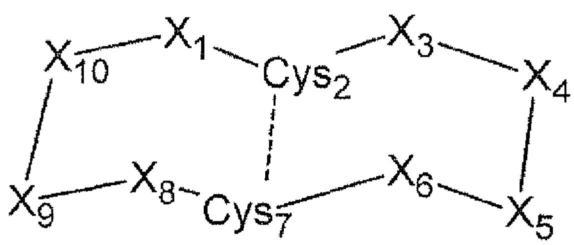

(Ia)

10. The use of the compounds of formula (I) according to any one of claims 1 to 9, for the preparation of a drug intended for the diagnosis, prevention and/or treatment of neurodegenerative diseases.

11. The use according to claim 10, for the treatment of Wilson's disease or for the treatment of Alzheimer's disease.

12. The use of the compounds of formula (I) according to any one of claims 1 to 9, for the preparation of a drug intended

for the diagnosis, prevention and/or treatment of poisoning with metal ions such as silver, cadmium, cobalt, copper, mercury, nickel, gold, lead and zinc ions.

**13.** The use according to claim 12, for the preparation of a drug intended for the treatment of poisoning with copper ions or mercury ions.

**14.** A pharmaceutical composition, **characterized in that** it comprises, as active ingredient, at least one compound of formula (I) according to any one of claims 1 to 9 and at least one pharmaceutically acceptable vehicle.

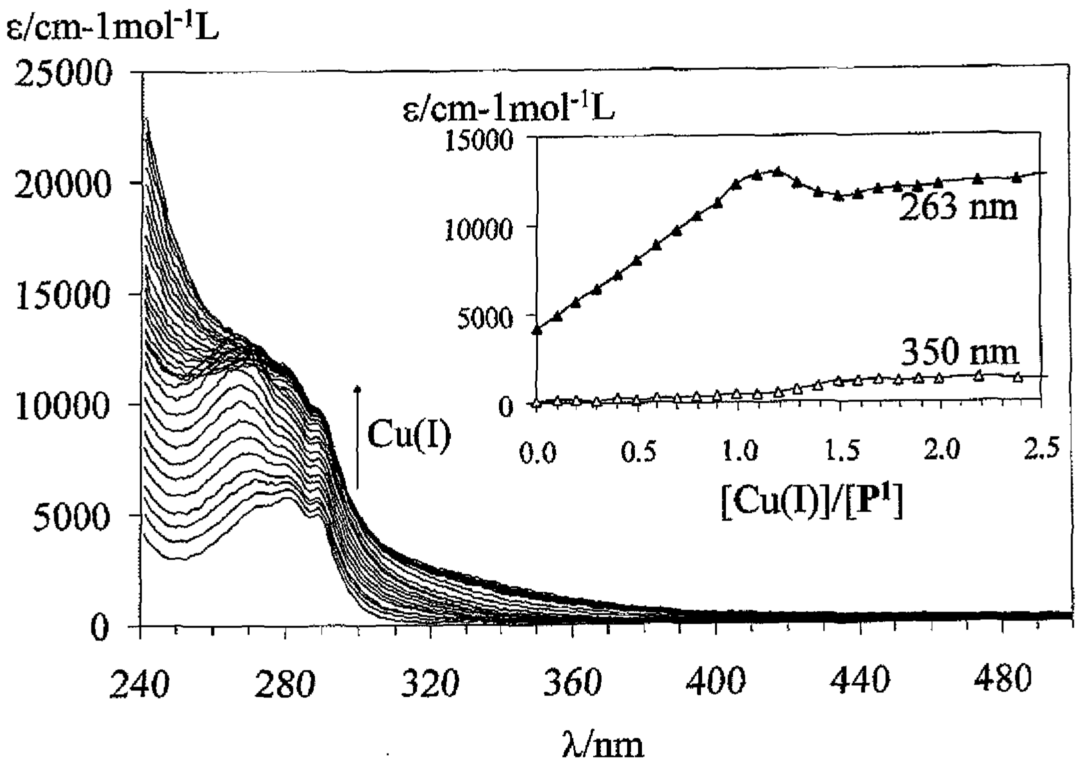
ε/cm-1mol-1L
25000
20000
15000
10000
5000
0
240
280
320
360
400
440
480
λ/nm
Cu(I)
ε/cm-1mol-1L
15000
10000
5000
0
0.0
0.5
1.0
1.5
2.0
2.5
[Cu(I)]/[P1]
263 nm
350 nm

FIGURE 1

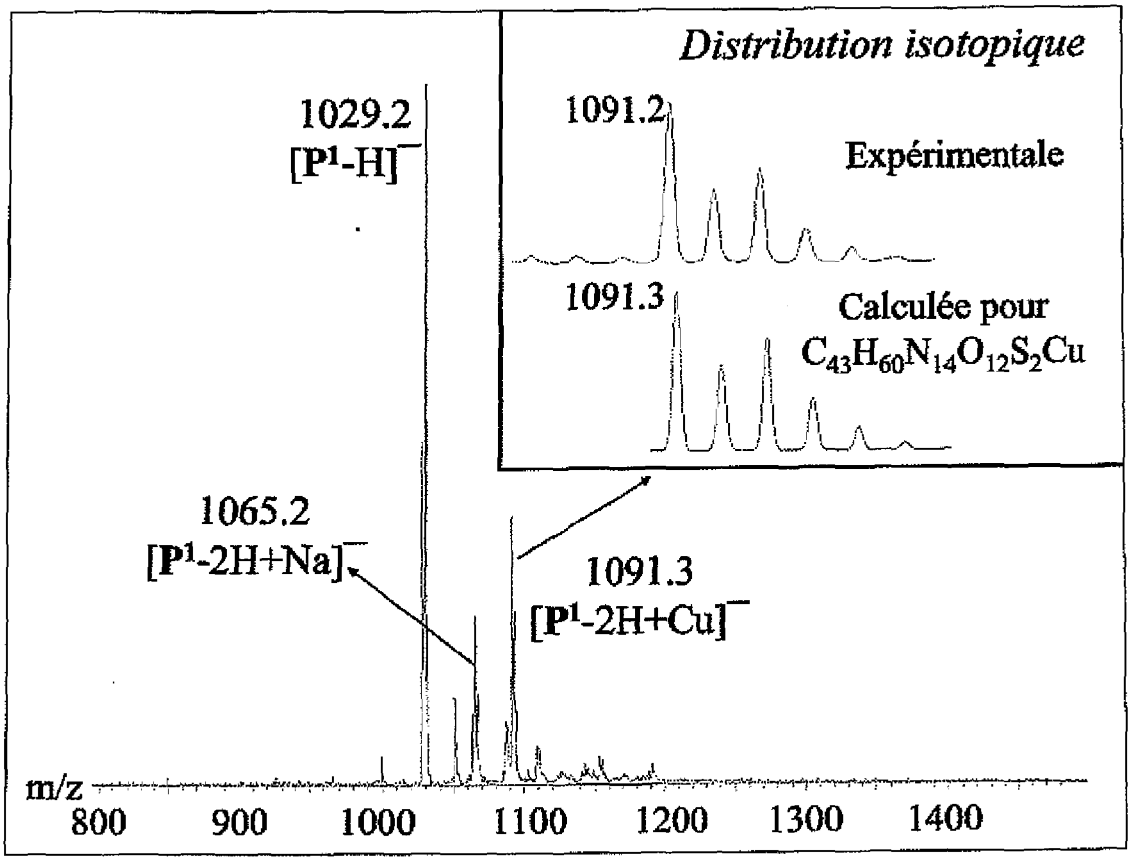

Distribution isotopique
1091.2
Expérimentale
1091.3
Calculée pour
C43H60N14O12S2Cu
1029.2
[P1-H]¯
1065.2
[P1-2H+Na]¯
1091.3
[P1-2H+Cu]¯
m/z
800
900
1000
1100
1200
1300
1400


FIGURE 2

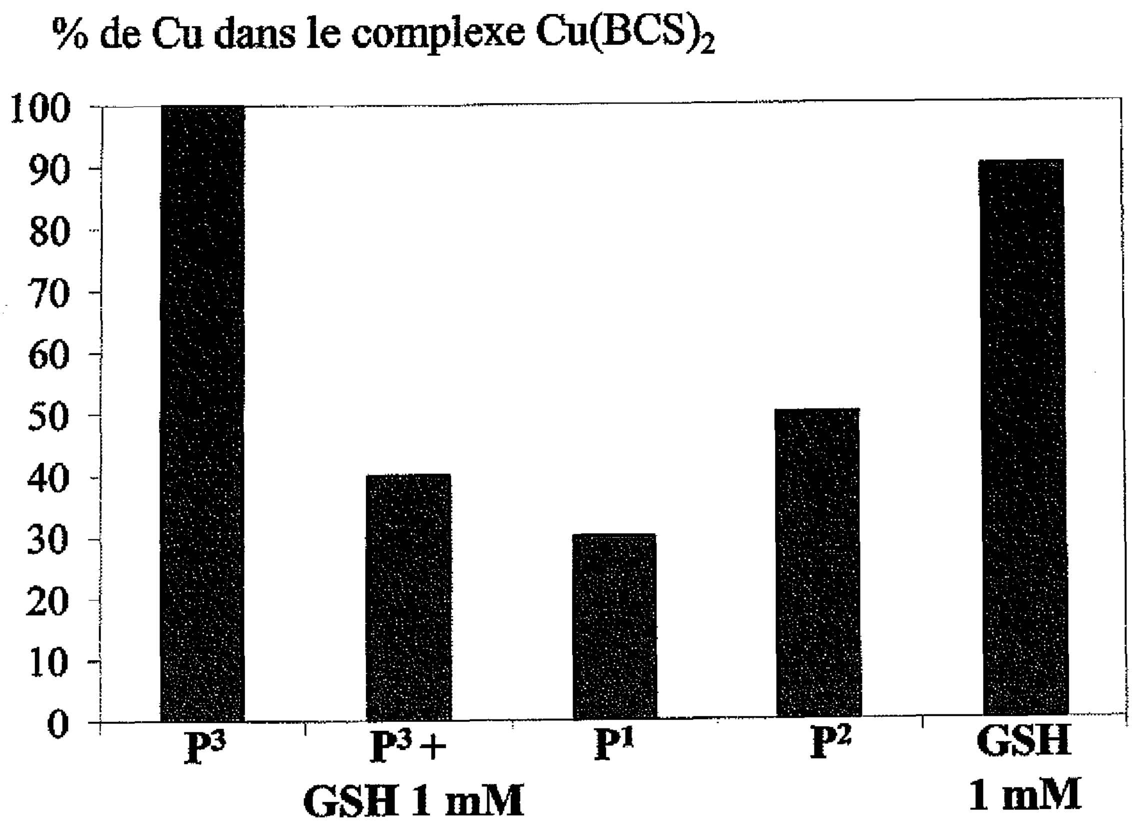
% de Cu dans le complexe Cu(BCS)2
100
90
80
70
60
50
40
30
20
10
0
P3
P3 +
GSH 1 mM
P1
P2
GSH
1 mM

FIGURE 3

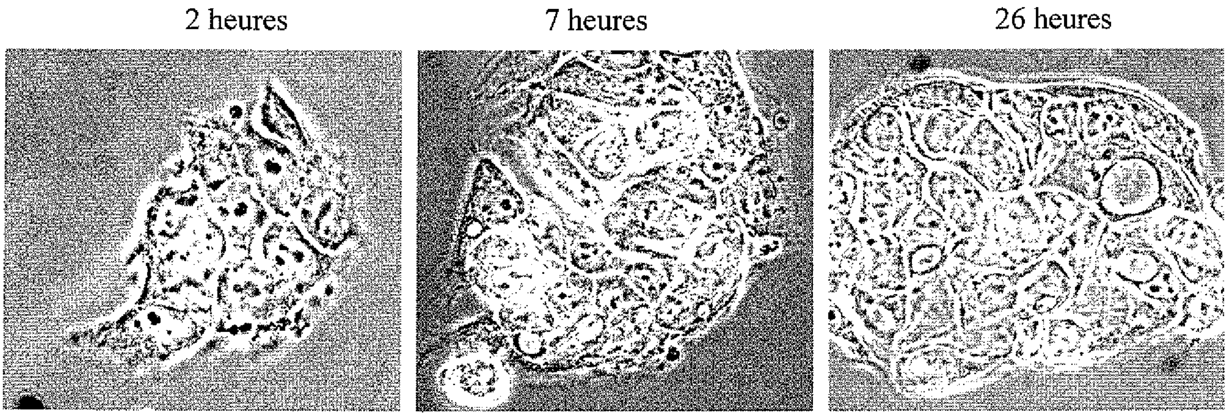


FIGURE 4

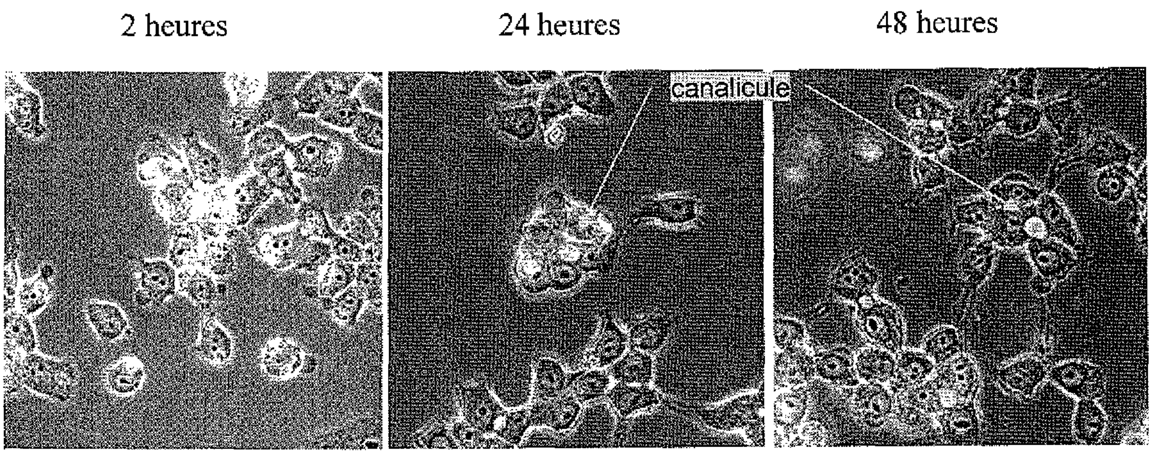


FIGURE 5

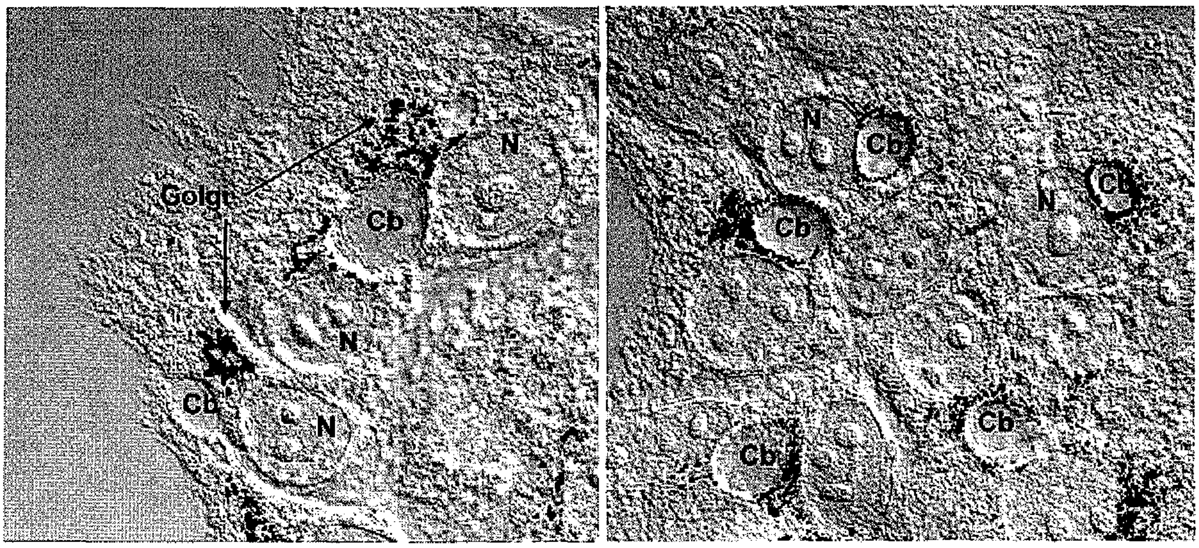


A B

FIGURE 6

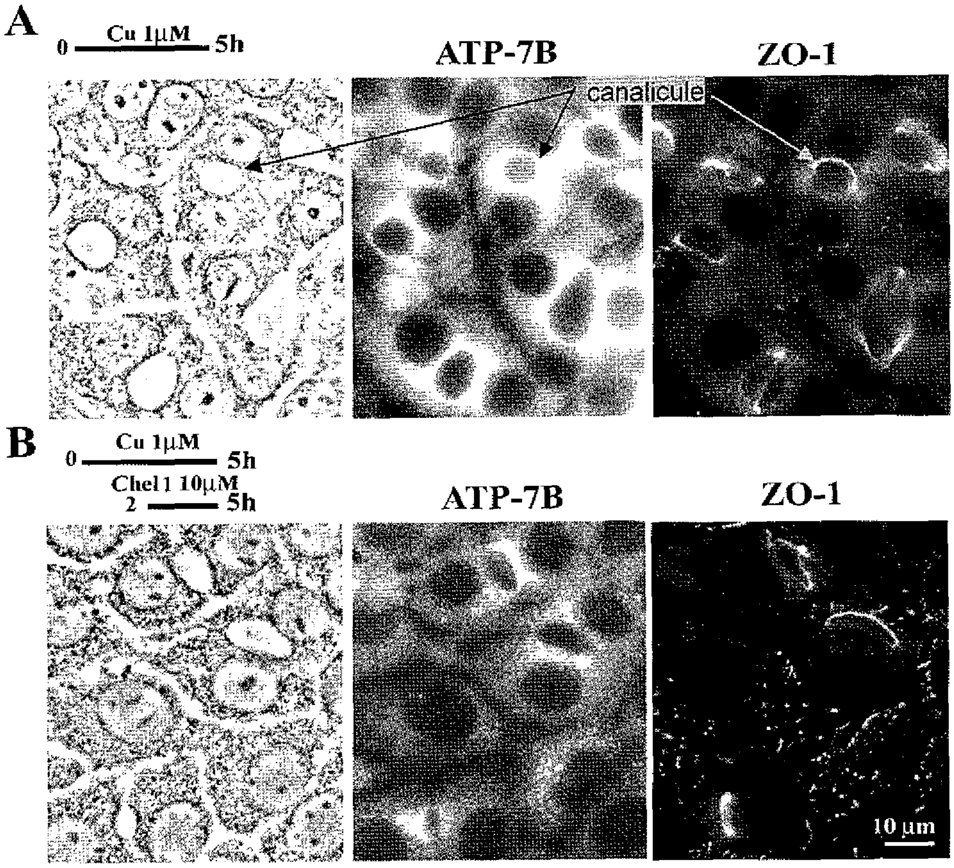
A
0 Cu 1µM 5h
ATP-7B
ZO-1
canalicule
B
0 Cu 1µM 5h
Chel1 10µM
2 5h
ATP-7B
ZO-1
10 µm

FIGURE 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **G. J. BREWER.** *DDT,* 2005, vol. 10, 1103-1109
- **M. HEFTER et al.** *J. C. S., Chem. Commun.,* 1993, 1704-1706
- **R. M. SMITH et al.** NIST Critically Selected Stability Constants of Metal Complexes Database. *NIST Standard Reference Database,* 2001, 46
- **B. SARKAR.** *Chem. Rev.,* 1999, vol. 99, 2535-2544
- **O. ANDERSEN.** *Chem. Rev.,* 1999, vol. 99, 2683-2710
- **G. J. BREWER et al.** *J. Hepatol.,* 2005, vol. 42, S13-S21
- **B. SARKAR.** *Chem. Rev.,* 1999, vol. 99, 2535-2534
- *Anti-Cancer Ag. in Med. Chem.,* 2008, vol. 8, 497-522
- *Cytometry,* 2006, vol. 69A, 863-871
- *Anal. Bioanal. Chem.,* 2006, vol. 384, 620-630
- **R. SHEPPARD.** *J. Peptide Sci.,* 2003, vol. 9, 545-552
- **RENAUDET et al.** *Org. Biomol. Chem.,* 2006, vol. 4, 2628-2636
- **S. FOUILLARD et al.** *J. ORG. Chem.,* 2008, vol. 73, 983-991
- **P. W. RIDDLES et al.** *Methods Enzymol.,* 1983, vol. 91, 49-60
- **Z. XIAO et al.** *J. Am. Chem. Soc.,* 2004, vol. 126, 3081-3090
- **P. ROUSSELOT-PAILLET et al.** *Inorg. Chem.,* 2006, vol. 45, 5510-5520
- **ROUSSELOT-PAILLET et al.** *Inorg. Chem.,* 2006, vol. 4, 2628-2635
- **C. DECAENS et al.** *J. Cell Sci.,* 1996, vol. 109, 1623-1635
- **X. PENG et al.** *Cell Tissue Res.,* 2006, vol. 323, 233-243
- **HERNANDEZ et al.** *Gastoenterology,* 2008, vol. 134, 1215-1223
- **Y. GUO et al.** *Am. J. Physiol. Gastrointest. Liver. Physiol.,* 2005, vol. 289, G904-G916